(19) Europäisches Patentamt
European Patent Office
Office européen des brevets

(11) **EP 4 278 980 B1**

(12) **EUROPEAN PATENT SPECIFICATION**

(45) Date of publication and mention of the grant of the patent:
**01.10.2025 Bulletin 2025/40**

(21) Application number: **22779004.5**

(22) Date of filing: **30.03.2022**

(51) International Patent Classification (IPC):
***A61B 8/02*** *(2006.01)* ***A61B 5/344*** *(2021.01)*
***A61B 5/1455*** *(2006.01)*

(52) Cooperative Patent Classification (CPC):
**A61B 5/1455; A61B 5/344; A61B 8/02; A61B 8/0866; A61B 8/0883; A61B 8/5223**

(86) International application number:
**PCT/CN2022/083998**

(87) International publication number:
**WO 2022/206822 (06.10.2022 Gazette 2022/40)**

(54) **FETAL DOPPLER AND DETECTION METHOD**

FÖTALER DOPPLER UND DETEKTIONSVERFAHREN

DOPPLER FOETAL ET PROCÉDÉ DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**02.04.2021 CN 202110362153**
**25.05.2021 CN 202110572668**
**03.12.2021 CN 202111467542**
**26.01.2022 CN 202220245322 U**
**26.01.2022 CN 202220225376 U**

(43) Date of publication of application:
**22.11.2023 Bulletin 2023/47**

(73) Proprietor: **Edan Instruments, Inc.**
**Shenzhen, Guangdong 518122 (CN)**

(72) Inventors:
- **CHEN, Dewei**
  **Shenzhen, Guangdong 518122 (CN)**
- **LIAO, Weita**
  **Shenzhen, Guangdong 518122 (CN)**
- **LIU, Jinqun**
  **Shenzhen, Guangdong 518122 (CN)**
- **YU, Junyu**
  **Shenzhen, Guangdong 518122 (CN)**
- **WANG, Gonghua**
  **Shenzhen, Guangdong 518122 (CN)**
- **WU, Yong**
  **Shenzhen, Guangdong 518122 (CN)**

(74) Representative: **J A Kemp LLP**
**80 Turnmill Street**
**London EC1M 5QU (GB)**

(56) References cited:
**CN-A- 102 485 180**
**CN-U- 206 183 300**
**CN-U- 206 355 073**
**KR-A- 20130 089 525**
**US-A1- 2011 218 413**
**US-A1- 2018 317 878**
**US-A1- 2021 059 538**
**US-B1- 6 340 346**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present disclosure relates to the technical field of medical devices, in particular relates to a fetal Doppler and a detection method.

Fetal heart-rate monitoring is a routine inspection item currently used for prenatal examination, and an ultrasonic fetal heart-rate instrument is usually used for detecting. Existing ultrasonic fetal heart-rate instruments, such as a fetal Doppler, may consider physiological data collected by its sensor as fetal physiological data, and the heart rate value calculated therefrom is directly used as a fetal heart rate, and another instrument is used to measure maternal parameters.

US 2018/0317878 A1 discloses a device and method for determining fetal heart rate.

In view of this, embodiments of the present disclosure provide a fetal Doppler and a detection method to solve problem of low detection efficiency of the existing instruments for maternal and fetal heart-rates.

According to a first aspect, embodiments of the present disclosure provide a fetal Doppler, comprising: a housing, and a fetal detection unit and a maternal parameter detection unit arranged on the housing, wherein the fetal detection unit and the maternal parameter detection unit are respectively used for collecting fetal physiological data and maternal physiological data; a controller is arranged in an internal cavity of the housing; the controller is used for analyzing the collected fetal physiological data and maternal physiological data, so as to obtain a fetal heart rate and a maternal parameter; wherein the fetal detection unit comprises an ultrasonic acquisition unit, and the ultrasonic acquisition unit comprises a wafer component (having a plurality of wafers and an angle adjustment component for adjusting a signal transmission direction between each of the wafers and a target body; the controller is connected to the angle adjustment component, and the controller is used for controlling the action of the angle adjustment component on the basis of the signal strength corresponding to each of the wafers, so as to realize target following.

According to a second aspect, the embodiments of the present disclosure also provide a detection method of a fetal Doppler, the fetal Doppler comprises a fetal detection unit and a maternal parameter detection unit, the detection method comprises: acquiring fetal physiological data collected by the fetal detection unit and maternal physiological data collected by the maternal parameter detection unit; analyzing the collected fetal physiological data and maternal physiological data to obtain a fetal heart rate and a maternal parameter; wherein the fetal detection unit comprises an ultrasonic acquisition unit, the ultrasonic acquisition unit comprises a plurality of wafers and an angle adjustment component; the step of acquiring the fetal physiological data collected by the fetal detection unit comprises: acquiring a signal strength corresponding to each of the wafers; based on the signal strength corresponding to each of the wafers, controlling an action of the angle adjustment component to realize target tracking, wherein the angle adjustment component is used for adjusting a signal transmission direction between each of the wafers and a target body; acquiring the fetal physiological data collected by each of the wafers based on the adjusted signal transmission direction.

According to the fetal Doppler provided by the embodiments of the present disclosure, the fetal detection unit and the maternal parameter detection unit are correspondingly used for collecting fetal physiological data and maternal physiological data, then the controller analyzes the fetal physiological data and the maternal physiological data respectively to obtain a fetal heart rate and a maternal parameter, so that it is realized that the maternal heart rate and the fetal heart rate are obtained by using one same fetal Doppler. As compared with using two instruments to obtain the fetal heart rate and the maternal parameter respectively, this solution can reduce the instrument cost by utilizing the same fetal Doppler, and since it can simultaneously obtain maternal and fetal parameters by directly using the same fetal Doppler, the instrument structure is simplified and the detection efficiency for maternal and fetal parameters is improved.

In order to illustrate technical solutions in specific embodiments of the present disclosure or in the prior art more clearly, drawings that need to be used in the description of the specific embodiments or the prior art are briefly introduced hereinafter. Apparently, the drawings described below are just some embodiments of the present disclosure. And for a person with ordinary skill in the art, other drawings can be obtained according to these drawings without expenditure of creative labor.

Figure 1 is a structural diagram of the fetal Doppler of an embodiment of the present disclosure;

Figure 2 is an overall structural schematic diagram of the fetal Doppler viewed from a first side in an embodiment of the present disclosure;

Figure 3 is a cross-sectional view of the fetal Doppler in an embodiment of the present disclosure;

Figure 4 is an enlarged view of the location A in Figure 3;

Figure 5 is an overall structural schematic diagram of the fetal Doppler viewed from a second side in an embodiment of the present disclosure;

Figure 6 is a structural schematic diagram of the ultrasonic acquisition unit in an embodiment of the present disclosure;

Figures 7a -7b are structural schematic diagrams of the wafer component in an embodiment of the present disclosure;

Figure 8 is a working principle schematic diagram of the ultrasonic acquisition unit in an embodiment of the present disclosure;

Figure 9 is a principle schematic diagram of angle adjustment in an embodiment of the present disclosure;

Figure 10 is a structural block diagram of the ultrasonic acquisition unit in an embodiment of the present disclosure;

Figure 11 is a frequency schematic diagram of an existing broadband receiving scheme;

Figure 12 is a frequency schematic diagram of a narrowband frequency-conversion receiving scheme in an embodiment of the present disclosure;

Figure 13 is a structural block diagram of a frequency conversion circuit in an embodiment of the present disclosure;

Figure 14 is another structural block diagram of a frequency conversion circuit in an embodiment of the present disclosure;

Figure 15 is a structural block diagram of the ultrasonic acquisition unit in an embodiment of the present disclosure;

Figure 16 is another structural block diagram of the ultrasonic acquisition unit in an embodiment of the present disclosure.

Figure 17 is a structural schematic diagram of an existing ultrasonic acquisition unit;

Figure 18 is a schematic diagram of the ultrasonic acquisition unit in an embodiment of the present disclosure;

Figures 19a - 19h are schematic diagrams of wafers in embodiments of the present disclosure;

Figure 20 is a spectrum schematic diagram in prior art;

Figure 21 is a spectrum schematic diagram that can be realized corresponding to Figure 18;

Figure 22 is a spectrum schematic diagram that can be realized corresponding to Figures 19a - 19d;

Figure 23 is a flowchart of the detection method of the fetal Doppler in an embodiment of the present disclosure;

Figure 24 is a control flowchart of the ultrasonic acquisition unit in an embodiment of the present disclosure;

Figure 25 is a flowchart of noise reduction for the fetal physiological data in an embodiment of the present disclosure;

Figure 26a - 26c are effect diagrams of signals after gain control in an embodiment of the present disclosure;

Figure 27 is a flowchart of acquiring the fetal physiological data in an embodiment of the present disclosure;

Figure 28 is a pulse signal schematic diagram in an embodiment of the present disclosure.

## Detailed description of the embodiments

In order to make objectives, technical solutions and advantages of embodiments of the present disclosure clearer, the technical solutions in the embodiments of the present disclosure will be described clearly and completely with reference to the drawings in the embodiments of the present disclosure. Apparently, the described embodiments are some embodiments of the present disclosure, rather than all of the embodiments. Based on the embodiments described in the present disclosure, all other embodiments obtainable by a person skilled in the art without expenditure of creative labor shall fall within the protection scope of the present disclosure.

The physiological data collected at an abdomen through a sensor by an existing ultrasonic fetal Doppler may be from a fetus or a mother, but because of being limited by ultrasonic principle, it is impossible to distinguish whether the collected physiological data is from a fetal heart beat or a pulse beat of the mother's main abdominal artery, an umbilical blood flow, etc., so that calculated heart rate value cannot be distinguished as a fetal heart rate or a maternal heart rate. Therefore, a general fetal Doppler would consider the collected physiological data as fetal physiological data, and directly take the heart rate value calculated therefrom as a fetal heart rate. Therefore, the existing ultrasonic fetal Doppler can only measure the fetal heart rate, and the maternal parameter needs to be measured by another instrument.

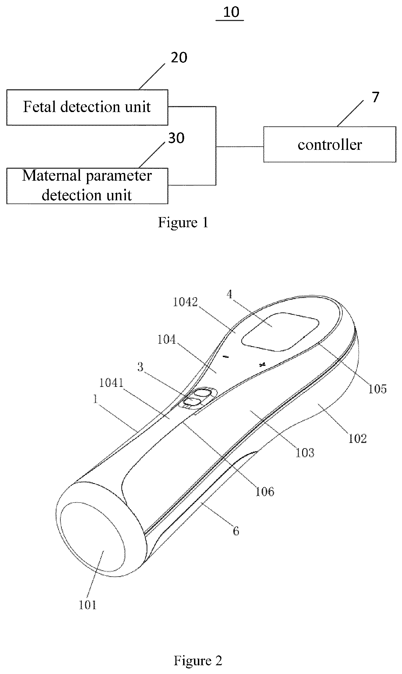

In view of this, the present disclosure provides a fetal Doppler, as shown in Figure 1, the fetal Doppler 10 comprises a controller 7, a fetal detection unit 20 and a maternal parameter detection unit 30 connected to the controller 7 respectively. The fetal detection unit 20 and the maternal parameter detection unit 30 are used for collecting fetal physiological data and maternal physiological data respectively. The controller 7 is used for analyzing the collected fetal physiological data and maternal physiological data to obtain a fetal heart rate and a maternal parameter.

It should be noted that the controller in embodiments of the present disclosure may only comprise one controller unit used for controlling the fetal detection unit and the maternal parameter detection unit, and analyzing the collected fetal physiological data and maternal physiological data. Alternatively, the controller comprises two controller units, one controller unit is used for controlling the fetal detection unit and the maternal parameter detection unit, and the other controller unit is used for analyzing the collected fetal physiological data and maternal physiological data. Alternatively, the controller comprises three controller units, one controller unit is used for controlling the fetal detection unit, another controller unit is used for controlling the maternal parameter detection unit, and yet another controller unit is used for analyzing the collected fetal physiological data and maternal physiological data.

The fetal physiological data may be data such as signal parameters etc. representing a fetal heart rate, and the maternal physiological data may be data such as signal parameters etc. representing a maternal heart rate. The fetal physiological data may be an ultrasonic echo signal acquired by a piezoelectric ceramic wafer, or other data representing the fetal heart rate. The maternal physiological data comprises, but not limited to, a pulse wave signal obtained by a photoelectric sensor, an ultrasonic echo signal obtained by a piezoelectric ceramic wafer, an ECG signal obtained by an ECG electrode, which may be a single type of signal or a mutually verified result of multiple types of signals, or it may be other data representing the maternal heart rate.

As compared with a fetal Doppler only used for monitoring a fetal heart-rate, the fetal Doppler 10 of the present disclosure comprises the fetal detection unit 20 and the maternal parameter detection unit 30, the fetal heart detection unit 20 and the maternal parameter detection unit 30 can respectively be used to correspondingly collect the fetal physiological data and the maternal physiological data, so as to realize that the maternal heart rate and the fetal heart rate are simultaneously obtained by using the same fetal Doppler 10, thereby achieving fetal heart-rate monitoring and maternal heart-rate monitoring simultaneously. As compared with using two instruments to acquire the fetal heart rate and the maternal parameter respectively, using one same fetal Doppler 10 can reduce the instrument cost. Wherein, the maternal parameter comprises, but not limited to, maternal heart rate and maternal blood oxygen, etc.

In some optional embodiments, when the fetal Doppler apparatus 10 uses the fetal detection unit 20 and the maternal parameter detection unit 30 to correspondingly collect the fetal physiological data and the maternal physiological data respectively, the fetal detection unit 20 may be configured to collect the fetal physiological data from a first position of a detection object, and the maternal parameter detection unit 30 may be configured to collect the maternal physiological data from a second position of the detection object. The first position and the second position may be same or different positions, as long as physiological data can be collected therefrom, and the specific position may be adjusted according to need. For example, in an application embodiment, the first position and the second position are different positions, the first position is an abdomen, and the second position is a hand, thus, the fetal physiological data may be collected from an abdomen of a detection object by using the fetal detection unit 20, and the maternal physiological data may be collected from a hand of the detection object by using the maternal parameter detection unit 30. The fetal detection unit 20 is used for collecting the fetal physiological data and may be a piezoelectric ceramic wafer; the maternal parameter detection unit 30 is used for collecting the maternal physiological data and may be a photoelectric sensor, a piezoelectric ceramic wafer or an ECG electrode, or at least one selected from a photoelectric sensor, a piezoelectric ceramic wafer and an ECG electrode. The fetal Doppler 10 may be an integral fetal heart-rate instrument, wherein a fetal detection unit 20, a maternal parameter detection unit 30 and a controller, comprising the controller 7, of the integral fetal heart-rate instrument are an integral body. In an application embodiment, the fetal Doppler 10 is an integral fetal heart-rate instrument, and its ultrasonic acquisition unit comprises a fetal detection unit 20 and a maternal parameter detection unit 30 arranged at different positions of the ultrasonic acquisition unit. Wherein, the fetal detection unit 20 is used for collecting the fetal physiological data from an abdomen of a detection object, and the maternal parameter detection unit 30 is used for collecting the maternal physiological data from a hand of the detection object. Although the fetal detection unit 20 and the maternal parameter detection unit 30 are in the same fetal Doppler, the fetal heart detection unit 20 acts on an abdomen of a detection object to collect the fetal physiological data from the abdomen, while the maternal parameter detection unit 30 acts on a hand of the detection object to collect the maternal physiological data from the hand. The detection object is a detected object of the fetal Doppler 10, such as a pregnant woman. In an application scenario, in order to achieve fetal heart-rate monitoring, a pregnant woman may hold the ultrasonic acquisition unit of the fetal Doppler 10 by herself to apply it to her abdomen, so that the fetal physiological data can be collected from the abdomen and the maternal physiological data can be collected from the hand, so that the fetal physiological data and the maternal physiological data may be monitored anytime and anywhere without being limited to special scenarios such as in hospitals.

When the fetal Doppler 10 uses the maternal parameter detection unit 30 to collect the maternal physiological data from a detection object or a second position of the detection object, the maternal parameter detection unit 30 may be a photoelectric sensor, and the maternal physiological data is a pulse wave signal. The pulse wave signal is a pulse wave signal that represents the maternal heart rate and is received by the photoelectric sensor after the photoelectric sensor transmits light with a preset wavelength to the second position of a pregnant woman, such as a finger pulp or a palm, which is rich in blood vessels. The light with the preset wavelength of the photoelectric sensor may be infrared light having a wavelength of 905 nm and/or red light having a wavelength of 660 nm. The pulse wave signal may be acquired by using the infrared light having a wavelength of 905 nm or the red light having a wavelength of 660 nm, when the infrared light having a wavelength of 905 nm and the red light having a wavelength of 660 nm are used together, blood oxygen saturation can be acquired. Therefore, in order to improve the accuracy of acquiring the maternal physiological data, the maternal parameter detection unit 30 may be a photoelectric sensor, and the maternal physiological data may be a pulse wave signal and blood oxygen saturation, that is, the photoelectric sensor emits the infrared light having a wavelength of 905 nm and the red light having a wavelength of 660 nm to obtain the maternal physiological data including the pulse wave signal and the blood oxygen saturation. Collecting the maternal physiological data by the photoelectric sensor would not cause the validity of maternal signal acquisition to be adversely affected by a short-circuit between ECG electrodes which may be caused by a coupling agent applied when using the ECG electrodes to collect the physiological data.

When the fetal Doppler 10 uses the maternal parameter detection unit 30 to collect the maternal physiological data from a detection object or a second position of the detection object, the maternal parameter detection unit 30 may be a piezoelectric ceramic wafer, and the maternal physiological data is an ultrasonic echo signal. The ultrasonic echo signal taken as the maternal physiological data is an ultrasonic echo signal that represents the maternal heart rate and is received by the piezoelectric ceramic wafer after the piezoelectric ceramic wafer transmits an ultrasonic pulse with a first preset frequency to a second position such as the hand of a detection object. Collecting the maternal physiological data by the piezoelectric ceramic wafer would not cause the validity of maternal signal acquisition to be adversely affected by a short-circuit between ECG electrodes which may be caused by a coupling agent applied when the ECG electrodes are used for collecting the physiological data. Because the fetus is located in the abdomen of a pregnant woman and thus the fetal physiological data cannot be collected by directly contacting the fetus. Therefore, when using the fetal detection unit 20 of the fetal Doppler 10 to collect fetal physiological data from a first position of a detection object, an ultrasonic principle is applied, the fetal detection unit 20 may be a piezoelectric ceramic wafer, and the fetal physiological data is an ultrasonic echo signal. The ultrasonic echo signal taken as the fetal physiological data is an ultrasonic echo signal representing the fetal heart rate and is received by the piezoelectric ceramic wafer after the piezoelectric ceramic wafer transmits an ultrasonic pulse with a second preset frequency to a first position of a detection object, such as an abdomen. The piezoelectric ceramic wafers used for collecting the maternal physiological data and the fetal physiological data may be one and the same, or may be plural and different. It can be understood that the sensor of the fetal Doppler 10 can directly act on the mother, but cannot directly act on the fetus. Therefore, as compared with the collection of fetal physiological data, the frequency of the piezoelectric ceramic wafer used for collecting maternal physiological data is higher. Therefore, in an application scenario, the fetal detection unit 20 and the maternal parameter detection unit 30 of the fetal Doppler 10 are both piezoelectric ceramic wafers, wherein one piezoelectric ceramic wafer collects maternal physiological data from a hand of a detection object, the first preset frequency of this piezoelectric ceramic wafer comprises but is not limited to 3M. The other piezoelectric ceramic wafer collects fetal physiological data from an abdomen of the detection object, the second preset frequency of this other piezoelectric ceramic wafer is 2M, 2.5M, etc. Frequency values of the first preset frequency and the second preset frequency may be custom set according to needs, and there is no specific limitation on them herein.

When the fetal Doppler 10 uses the maternal parameter detection unit 30 to collect the maternal physiological data from a detection object or a second position of the detection object, the maternal parameter detection unit 30 may be ECG electrodes, and the maternal physiological data is an ECG signal. The ECG signal taken as the maternal physiological data may be an ECG signal obtained by the ECG electrodes from a second position of a pregnant woman, such as between different fingers, between fingers and a palm, between limbs and an abdomen, etc. The ECG electrodes may comprise a plurality of electrode pieces, and the ECG signal is obtained by measuring a potential difference between different electrode pieces acting on different positions. Taking the ECG electrodes comprising two electrode pieces as an example, when the ECG electrodes obtain an ECG signal between different fingers, the two electrode pieces act on different fingers respectively; when the ECG electrodes obtain an ECG signal between a finger and a palm, one electrode piece acts on the finger and the other electrode piece acts on the palm; when the ECG electrodes obtain an ECG signal between a limb and a abdomen, one electrode piece acts on the limb such as acting on a finger or a palm, and the other electrode piece acts on the abdomen, at this time, because the distance between the acting positions of the two electrode pieces are relatively far, the potential difference is large, and the ECG signal can reflect the maternal physiological data more accurately. As compared with adding an ECG electrode outside the ultrasonic acquisition unit and acquiring the maternal heart rate at the abdomen of a pregnant woman, wherein, because a coupling agent needs to be used during the process of using the ultrasonic acquisition unit and the coupling agent belongs to a conductor, a short-circuit between different electrodes is easily caused, which adversely affects the use of the ECG electrode, and a disposable ECG electrode would increase the use cost of the fetal Doppler 10, while the metal electrode is vulnerable to corrosion by the coupling agent, which adversely affects the validity of maternal physiological data acquisition, in contrast, in this embodiment, when the maternal parameter detection unit 30 comprises ECG electrodes and the object of action when collecting the maternal physiological data is a hand of a detection object, the use of the coupling agent is reduced, thereby reducing the adverse influence of the short-circuit between the ECG electrodes caused by the coupling agent.

In this embodiment, the same fetal Doppler 10 can collect both the fetal physiological data and the maternal physiological data, so that the maternal heart rate and fetal heart rate can be obtained by using the same device. As compared with using an ultrasonic acquisition unit and a uterine contraction pressure ultrasonic acquisition unit simultaneously for acquiring the fetal physiological data and the maternal physiological data respectively, in this embodiment, the fetal physiological data and the maternal physiological data are acquired simultaneously by using the fetal Doppler 10, which is easy to use and convenient to operate. In addition, by using the same fetal Doppler 10, the instrument cost can be reduced, and the instrument structure can be simplified. As compared with the prior art that the maternal parameter detection unit 30 is added into a uterine contraction pressure ultrasonic acquisition unit to obtain the maternal heart rate, in this disclosure, two detection units are used directly on the same device, and no additional ultrasonic acquisition unit is needed for adding the maternal parameter detection unit 30 therein, which also reduces the inconvenience of using a plurality of ultrasonic acquisition units for a pregnant woman. Meanwhile, as compared with the prior art that an external maternal ECG electrode is connected outside the uterine contraction pressure ultrasonic acquisition unit to obtain the maternal heart rate, the technical solution of the present disclosure adds a maternal parameter detection unit 30 on the fetal Doppler for measuring the maternal heart rate. The fetal Doppler 10 is an integral household fetal Doppler, it is generally used for measuring the fetal heart rate. However, due to the limitation of Doppler principle during the measuring process, it may be mixed with signals such as maternal cardiac pulse, maternal abdominal aorta pulse or umbilical blood flow, and in addition, due to a pregnant woman or others is lacking of experience, the real fetal heart rate is unable to be identified in domestic use. Therefore, in the present disclosure, by arranging the maternal parameter detection unit 30 in the fetal Doppler for measuring and processing the maternal physiological data, it is convenient for a pregnant women and other detection objects to view the real fetal heart rate. A detection object can monitor the maternal heart rate without using a uterine contraction pressure ultrasonic acquisition unit and an ultrasonic acquisition unit at the same time. Moreover, the maternal parameter detection unit 30 is provided on the same instrument, which will reduce instrument cost. As compared with the prior art wherein the ECG electrode requires the use of an ultrasonic coupling agent which will cause the disposable ECG electrode to be corroded by the coupling agent, in the present disclosure, the maternal parameter detection unit for collecting maternal physiological data may be a photoelectric sensor or a piezoelectric ceramic wafer, which would not cause the validity of maternal signal acquisition to be adversely affected by a short-circuit between ECG electrodes due to the coupling agent.

The controller 7 of the fetal Doppler 10 may analyze the fetal physiological data and the maternal physiological data respectively to obtain the fetal heart rate and the maternal parameter. The details are as follows:

The maternal-fetal heart rates may comprise a maternal heart rate that representing a maternal pulse rate value and a fetal heart rate that representing a fetal pulse rate value. After obtaining the fetal physiological data and the maternal physiological data by using the fetal detection unit 20 and the maternal parameter detection unit 30 of the fetal Doppler 10, the fetal physiological data may be analyzed to obtain the fetal heart rate, and the maternal physiological data is analyzed to obtain the maternal heart rate. The method for analyzing the fetal physiological data and the maternal physiological data may be any algorithm that is capable of implementing the analysis of the fetal physiological data and the maternal physiological data, it is not specifically limited herein.

When the controller 7 analyzes the maternal physiological data to obtain the maternal heart rate, according to different ways of collecting the maternal physiological data by the fetal detection unit 20 and the maternal parameter detection unit 30 of the fetal Doppler 10, for example, according to different types of the maternal parameter detection unit 30, different processing modes are used for analyzing to obtain the maternal heart rate. In an embodiment, the maternal parameter detection unit 30 is a photoelectric sensor, and the maternal physiological data is a pulse wave signal, thus, when the controller 7 analyzes the maternal physiological data to obtain the maternal heart rate, the controller 7 is further used for determining an envelope of the pulse wave signal, and obtaining the maternal heart rate based on the envelope. If the maternal parameter detection unit 30 is a photoelectric sensor, the maternal physiological data is a pulse wave signal and blood oxygen saturation, the controller 7 is further used for obtaining the maternal heart rate based on the envelope of the pulse wave signal and the blood oxygen saturation. In another embodiment, the maternal parameter detection unit 30 is a piezoelectric ceramic wafer, and the maternal physiological data is an ultrasonic echo signal, thus, when the controller 7 analyzes the maternal physiological data to obtain the maternal heart rate, the maximum frequency envelope signal in the ultrasonic echo signal may be acquired, and the maternal heart rate is obtained based on the maximum frequency envelope signal. In yet another embodiment, the maternal parameter detection unit 30 comprises ECG electrodes, and the maternal physiological data is an ECG signal, thus, when the controller 7 analyzes the maternal physiological data to obtain the maternal heart rate, the ECG signal may be analyzed to obtain the maternal heart rate.

When the fetal heart detection unit 20 is a piezoelectric ceramic wafer, the fetal physiological data is an ultrasonic echo signal, thus, when the controller 7 analyzes the maternal physiological data to obtain the fetal heart rate, the controller 7 may be used for acquiring the maximum frequency envelope signal in the ultrasonic echo signal, and obtaining the fetal heart rate based on the maximum frequency envelope signal.

As compared with using two instruments to obtain the fetal heart rate and the maternal heart rate respectively, the present disclosure can reduce the instrument cost by utilizing the same fetal Doppler, and by directly using the fetal detection unit and the maternal parameter detection unit on the same fetal Doppler to obtain maternal and fetal heart rates, the instrument structure can be simplified.

An embodiment of the present disclosure provides a fetal Doppler, the maternal parameter detection unit thereof is a blood oxygen acquisition unit, and the fetal detection unit thereof is an ultrasonic acquisition unit. As shown in Figure 2, the fetal Doppler comprises a housing 1, an ultrasonic acquisition unit 2, and a blood oxygen acquisition unit 3; the fetal Doppler is specifically a handheld fetal Doppler. The ultrasonic acquisition unit 2 is arranged on one end of the housing 1 for detecting the fetal heart-rate signal of a detection object; the blood oxygen acquisition unit is arranged inside the housing 1 for detecting a blood oxygen signal of the detection object. This fetal Doppler with the blood oxygen acquisition function may realize the multi-purpose usage of the fetal Doppler, and it is more convenient to carry; when the fetal Doppler is used in home environment, waste of resources caused by abandonment of the fetal Doppler after the birth of the fetus is avoided.

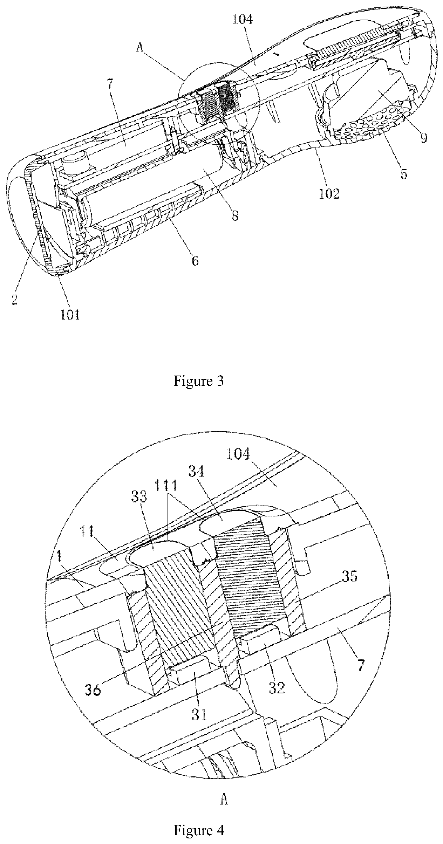

As shown in Figures 1-3, the housing 1 is provided with a light transmitting window 111, and the blood oxygen acquisition unit is equipped with a light guide structure installed on the light transmitting window 111. The controller 7 is arranged in an internal cavity of the housing 1. The blood oxygen acquisition unit 3 and the ultrasonic acquisition unit 2 are electrically connected to the controller 7. The blood oxygen acquisition unit comprises a light emitting element 31 for emitting light and a light receiving element 32 for receiving light that are both arranged on the controller 7, and the light guide structure comprises a first light guide member installed on the light transmitting window 111; the first light guide member is located on a light transmission path between the light emitting element 31 and the light transmitting window 111, and is configured to transmit light emitted by the light emitting element 31 to the outside of the light transmitting window 111 after being propagated by the first light guide member.

By providing the first light guide member on the light transmission path between the light emitting element 31 and the light transmitting window 111, the light emitted by the light emitting element 31 can be better transmitted to the outside of the light transmitting window 111 after being propagated by the first light guide member. As compared with a blood oxygen measurement scheme that is not provided with the first light guide member, the light emitted by the light emitting element 31 can be more effectively transmitted to the surface of an object to be measured, and it can also realize accurate measurement of blood oxygen parameter in case of a relatively long measurement distance; and as compared with a blood oxygen measurement scheme by externally connecting a blood oxygen ultrasonic acquisition unit by a wire, the wire cost can be reduced, and a valid signal can also be collected at a lower cost.

In some optional embodiments, the light guide structure further comprises a first barrier arranged in the housing 1 and located between the light transmitting window 111 and the controller 7, wherein a first optical path channel is provided inside the first barrier, the first light guide member is a first light guide column 33 arranged in the first optical path channel. The first light guide column 33 is a transparent cylindrical glass light guide column, and the first barrier is made of a material having good light shielding performance and good internal surface reflectivity. The first barrier can prevent the emitted light from being transmitted through it, and reflect the emitted light incident to the inner surface of the first barrier back into the first light guide member for propagation, thereby reducing leakage of the emitted light in the housing 1, and enabling more emitted light to be emitted outward through the first light guide member, and improving light intensity of the light emitted from the light transmitting window 111, which is conducive to improving accuracy of blood oxygen measurement. In some other embodiments, the shape of the first light guide member may also not be limited to a cylindrical shape, and the first light guide member may be an integral structure or a combined structure composed of a plurality of light guide members, as long as the first light guide is conducive to focusing the emitted light emitted by the light emitting element 31 in one direction to be transmitted through the light-transmitting window 111.

In some optional embodiments, the light guide structure further comprises a second light guide member arranged in the housing 1 and located on a light transmission path between the light receiving element 32 and the light transmitting window 111, and the second light guide member is configured to transmit light incident from the light transmitting window 111 to the light receiving element 32 after being propagated by the second light guide. The second light guide member can transmit the light reflected from the surface of a detection object to the light receiving element 32 more effectively, which is conducive to collecting valid signals by the light receiving element 32 and improving accuracy of the blood oxygen measurement result. Further, there is a second barrier arranged in the housing 1 and located between the light transmitting window 111 and the controller 7, a second optical path channel is provided inside the second barrier, and the second light guide member is a second light guide column 34 arranged in the second optical path channel. Specifically, the structures, shapes and materials of the second light guide column 34 and the first light guide column 33 are the same; the materials of the second barrier and the first barrier are also the same. The second barrier can prevent the light from being transmitted through it, and reflect the light incident to the inner surface of the second barrier back into the second light guide member for propagation, thereby reducing leakage of incident light in the housing 1, and enabling more incident light to be transmitted to the light receiving element 32 through the second light guide member, which is conducive to improving light intensity of the incident light received by the light receiving element 32 and improving accuracy of blood oxygen measurement.

In some optional embodiments, the first barrier and the second barrier are integrally formed by a blocking base 35, the first optical path channel is a first through hole provided in the blocking base 35, the second optical path channel is a second through hole provided in the blocking base 35. The integral design of the first barrier and the second barrier has advantages of a simple structure, few parts, easy assembly and low cost. In alternative embodiments, the first barrier and the second barrier may also be two independent columnar structures each having a through hole therein.

Further, the controller 7 is provided with a notch located between the light emitting element 31 and the light receiving element 32, the blocking base 35 is provided with a plugboard 36 inserted into the notch, the plugboard 36 is located between the first through hole and the second through hole. The plugboard 36 can not only prevent light from leaking through a gap between the blocking base 35 and the controller 7, but also prevent a crosstalk-of-light phenomenon between the light emitting element 31 and the light receiving element, which is conducive to improving accuracy of blood oxygen measurement.

In some optional embodiments, the light emitting element 31 and the first through hole of the blocking base 35 are arranged to directly face each other, the light receiving element 32 and the second through hole of the barrier seat 35 are arranged to directly face each other, that is, the light emitting element 31 and the light receiving element 32 are both located directly under the light transmitting window 111. The light emitting element 31, the first light guide column 33 and the light transmitting window 111 are arranged along the same straight line, and light emitted from the light emitting element 31 can be emitted rectilinearly outward through the light transmitting window 111, thereby reducing loss of the emitted light; the light receiving element 32, the second light guide column 34 and the light transmitting window 111 are arranged along the same straight line, and the reflected light incident through the light transmitting window 111 can be received by the light receiving element 32 rectilinearly through the second light guide column 34, which is conducive to improving light intensity of the incident light received by the light receiving element 32 and improving accuracy of blood oxygen measurement. In some other embodiments, the light emitting element 31 and the light receiving element 32 may not be arranged directly under the light transmitting window 111, and light emitted from the light emitting element 31 is transmitted to the outside of the light transmitting window 111 after being refracted, and the reflected light incident through the light transmitting window 111 is received by the light receiving element 32 after being refracted; in this way, the opening position of the light transmitting window 111 on the housing 1 would not be restricted by the positions of the light transmitting element 31 and the light receiving element 32 on the controller 7, and thus the light transmitting window 111 can be arranged at any suitable position on the housing 1 for facilitating operation.

In some optional embodiments, the housing 1 is connected to a cover plate 11 located above the blocking base 35, two openings provided in the cover plate 11 form the light transmitting window 111, a lower end surface of the cover plate 11 abuts against an upper end face of the blocking base 35, the first light guide column 33 and the second light guide column 34 are each provided with a stepped limiting surface at the same height as the upper end face of the blocking base 35, the lower end surface of the cover plate 11 abuts against the stepped limiting surfaces on the first light guide column 33 and the second light guide column 34. In this way, it is convenient to install the blocking base 35, the first light guide column 33 and the second first light guide column 34 inside the housing 1 in sequence, and then the blocking base 35, the first light guide column 33 and the second light guide column 34 can be position-limited by the lower end surface of the cover plate 11, so that the first light guide column 33 and the second light guide column 34 can be stably fixed on the housing 1 to prevent the first light guide column 33 and the second light guide column 34 from falling off.

In some optional embodiments, the first light guide member is a condensing lens that is capable of converging light, and the condensing lens can enable the focus point of emitted light to just fall on the surface of a detection object, and can transmit a light detection signal having larger light intensity to the detection object. Specifically, the condensing lens comprises a convex lens, one surface of the convex lens at a side facing the light emitting element 31 is a convex surface. In an alternative embodiment, the condensing lens may also be a condensing lens group composed of one or more convex lenses or planar lenses.

By arranging a blocking base having a first through hole between the light transmitting window 111 and the light emitting element 31 as well as a second through hole between the light transmitting window 111 and the light receiving element 32, arranging a first light guide column 33 in the first through hole of the blocking base, and arranging a second light guide column 34 in the second through hole of the blocking base, emission light emitted by the light emitting element 31 can be transmitted to the surface of a detection object more effectively, reflection light reflected from the surface of the detection object can also be received by the light receiving element 32 more effectively, so that accurate measurement of the blood oxygen parameter can be realized at lower cost and a longer measurement distance.

In some optional embodiments, the fetal detection unit comprises an ultrasonic acquisition unit, as shown in Figures 2-5, the fetal Doppler further comprises a display screen 4, a loudspeaker 9 and a battery 8. The ultrasonic acquisition unit 2, the blood oxygen acquisition unit 3, the loudspeaker 9, the controller 7 and the battery 8 are all arranged in the inner cavity of the housing 1.

The housing 1 is in a long columnar shape. The housing 1 includes an end face housing 101 and a columnar housing. The columnar housing is connected to one side of the end face housing 101. The ultrasonic acquisition surface of the ultrasonic acquisition unit 2 is arranged to correspond to the position of the end face housing 101; the blood oxygen acquisition surface of the blood oxygen acquisition unit 3 is located on the columnar housing and is exposed outwards from the columnar housing. For this kind of fetal Doppler having blood oxygen measuring function, the ultrasonic acquisition surface of the ultrasonic acquisition unit 2 and the blood oxygen acquisition surface of the blood oxygen acquisition unit 3 are located on two different surfaces of the housing 1. When a detection object holds the fetal Doppler with hand and points the ultrasonic acquisition surface towards the detection object itself, the ultrasonic acquisition unit 2 can detect the fetal heart-rate signal of the detection object. At the same time, because the hand of the detection object holds the blood oxygen acquisition surface located on the columnar housing, the blood oxygen acquisition unit 3 can simultaneously detect a blood oxygen signal of the detection object, so that the fetal heart-rate signal and the blood oxygen signal of the detection object can be detected simultaneously. As compared with the mode of collecting blood oxygen signal by using a blood oxygen detection clamp in the prior art, the fetal Doppler does not need to use a blood oxygen detection clamp for clamping a finger, and the operation is more convenient. In addition, a wire required for connecting a blood oxygen detection clamp to the fetal Doppler is also omitted, which can reduce the product cost. In some other embodiments, the housing 1 may also have an elliptical shape, as long as the shape of the housing 1 is convenient to hold with hand and the ultrasonic acquisition surface of the ultrasonic acquisition unit 2 and the blood oxygen acquisition surface of the blood oxygen acquisition unit 3 are located on two different surfaces of the housing 1.

In some optional embodiments, the columnar housing comprises a planar housing part 104 and a curved housing part. There are two pieces of the curved housing part, namely a first curved housing piece 102 and a second curved housing piece 103. The first curved housing piece 102 and the second curved housing piece 103 are interconnected to form an inner cavity of the housing 1, and the first curved housing piece 102 and the second curved housing piece 103 are both connected to one side of the end face housing 101; the top of the second curved surface housing piece 103 is provided with a mounting groove, the planar housing part 104 is embedded into the mounting groove, and the upper top surface of the planar housing part 104 is flush with the groove mouth of the mounting groove. The blood oxygen acquisition surface of the blood oxygen acquisition unit 3 is located on the planar housing part 104 and exposed outwards from the planar housing part 104. Because the columnar housing comprises the curved housing part and the planar housing part 104, the curved housing part has better adaptability to a hand shape in the holding and folding state, and the hand feeling is better for using; and the planar housing part 104 facilitates installing the blood oxygen acquisition unit 3 on the housing 1, and facilitates collecting a valid blood oxygen signal by the blood oxygen acquisition unit 3. It should be noted herein that, the planar housing part 104 does not specifically refer to that the surface of the planar housing part 104 is completely planar, and it is only necessary that a portion of the planar housing part 104 is planar for it to be identified as the planar housing part 104 described in this embodiment; similarly, the curved housing part does not specifically refer to that the outer surface of the curved housing part is completely curved, but it is only required that a local shape of the curved housing part adapts to the hand shape in the holding and folding state.

A boundary between the planar housing part 104 and the curved housing part is an arc. The arc comprises a convex arc section 105 protruding outward and a concave arc section 106 concaving inward, a portion of the planar housing part 104 enclosed by two concave arc sections 106 is an inward contraction region 1041. a portion of the planar housing part 104 enclosed by the convex arc sections 105 is an outward expansion region 1042. The outward expansion region 1042 is located at an end of the inward contraction region 1041 away from the columnar housing. The blood oxygen acquisition surface of the blood oxygen acquisition unit 3 is arranged at a side of the inward contraction region 1041 adjacent to the outward expansion region 1042. The blood oxygen acquisition surface is located at a narrow position in the middle of the planar housing part 104. When holding the housing 1 with a hand, a contact position between the thumb of the hand and the housing 1 may be just on the blood oxygen acquisition surface, which is conducive to collecting a blood oxygen signal. In some other embodiments, the boundary between the planar housing part 104 and the curved housing part may also be a straight line, and the blood oxygen acquisition surface of the blood oxygen acquisition unit 3 is arranged at a middle position in the length direction of the planar housing part 104.

The outward expansion region 1042 of the planar housing part 104 is provided with a display screen 4, the display screen 4 is installed on the second curved housing piece 103. An area of the planar housing part 104 corresponding to the display screen 4 is provided with a square hole, the part of the display screen 4 protruding from the second curved housing piece 103 is embedded into the square hole of the planar housing part 104, and an upper end surface of the display screen 4 is flush with an upper end surface of the planar housing part 104. Because the display screen 4 is arranged on the outward expansion region 1042 of the planar housing part 104, the outward expansion region 1042 can provide a sufficiently large display space, thereby facilitating the installation of a display module having a large size, and facilitating viewing the measurement result displayed by the display screen 4.

In some optional embodiments, the first curved housing piece 102 is provided with speaker holes 5 corresponding to the position of the loudspeaker 9, and the first curved housing piece 102 is further provided with a battery cover 6 covered on the outer side of the battery 8. The speaker holes 5 and the battery cover 6 are located on one surface of the second curved housing piece 103 at a side facing away from the planar housing 104. Because the speaker holes 5 and the battery cover 6 are arranged on one side of the second curved housing 103 facing away from the planar housing part 104, the installation space on the side of the plane housing part 104 would not be occupied, thereby realizing the miniaturization and compact design of the fetal Doppler.

In some embodiments, the blood oxygen acquisition unit 3 comprises a blood oxygen measurement module for acquiring a blood oxygen signal, a control module electrically connected to the blood oxygen measurement module, and a trigger sensing module arranged on the blood oxygen acquisition surface and electrically connected to the control module. When the trigger sensing module senses that there is an obstructing object near the blood oxygen acquisition surface, the trigger sensing module sends a control signal to the control module for controlling the blood oxygen measurement module to start. In this way, automatic detection of a blood oxygen signal can be realized, and the blood oxygen signal can be detected at the same time of detecting the fetal heart-rate signal, and the competitiveness of the product is improved.

In some optional embodiments, the ultrasonic acquisition unit performs sound source locating of the fetal position by means of signal strength of each wafer, and realizes target tracking in the case of fetal movement, so as to reduce risks of failure of the ultrasonic acquisition unit to obtain a valid fetal signal due to fetal movement, body position change and other factors in the process of fetal heart-rate interaction that results in discontinuous fetal heart-rate signal acquisition and abnormal calculation of fetal heart rate, etc.

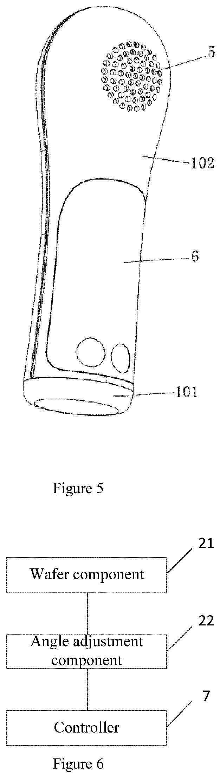

As shown in Figure 6, the ultrasonic acquisition unit comprises a wafer component 21 and an angle adjustment component 22. Wherein, the wafer component 21 and the angle adjustment component 22 are both connected to the controller. During the operation of the ultrasonic acquisition unit, the controller is used for controlling action of the angle adjustment component 22 based on signal strength corresponding to each wafer to realize target tracking.

Specifically, the wafer component 21 comprises a plurality of wafers, and there is no restriction on the specific number and arrangement manner of the wafers, which may be specifically configured according to the actual needs. Wherein, each wafer may be a complete wafer that transmits an ultrasonic signal in a pulse wave mode; the ultrasonic signal may also be transmitted in a continuous wave mode, that is, at the position of one wafer, a complete wafer is composed of an ultrasonic transmitting half-wafer and an ultrasonic receiving half-wafer.

The angle adjustment component 22 may be used for adjusting the position of the wafer component 21, and may also be used for adjusting an ultrasonic emission direction of each wafer in the wafer component 21, etc. When the angle adjustment component 22 is used for adjusting the position of the wafer component 21, the controller determines an action parameter of the angle adjustment component 22 by using signal strength corresponding to each wafer, and controls the action of the angle adjustment component 22 based on the action parameter, and accordingly, the position of the wafer component can be adjusted. When the position of the wafer component is changed, a relative position between the wafer component and a target body is changed, so that a signal transmission direction between each wafer and the target body can be realized.

The angle adjustment component 22 may also adjust the ultrasonic emission directions of the wafers without changing the position of the wafer component. For example, a propagation direction of an ultrasonic signal may be adjusted by using the working principle of a reflector, and thus the ultrasonic emission direction of the wafer is adjusted; that is, under the condition that the position of the wafer component is not changed, the target tracking is realized by changing the ultrasonic propagation path.

The controller may be a single chip microcomputer, a FPGA or other programmable logic controllers, and may be specifically configured according to actual needs. The controller receives a signal corresponding to each wafer, and processes the received signal to obtain the corresponding signal strength, and then determines an action for the angle adjustment component 22 based on the signal strength of each wafer to realize target tracking.

The signal transmission direction between the wafer and the target body is adjusted by the angle adjustment component, wherein the action of the angle adjustment component is controlled based on signal strength corresponding to each wafer, that is, the wafer component can always follow the target in a target tracking mode to obtain an accurate fetal heart-rate signal. That is, the ultrasonic acquisition unit can automatically follow the target, thereby avoiding manual adjustment.

In some optional embodiments, the plurality of wafers in the wafer component 21 may be divided into central wafers and peripheral wafers according to their arrangement positions. Wherein, the peripheral wafers are arranged around a periphery of the central wafers.

The plurality of wafers are divided into central wafers and peripheral wafers to realize multi-directional detection, and then an action of the angle adjustment component is controlled by means of signal strength corresponding to the peripheral wafers and the central wafers, that is, the action of the angle adjustment component is controlled on the basis of multi-directional detection to ensure a better target tracking effect.

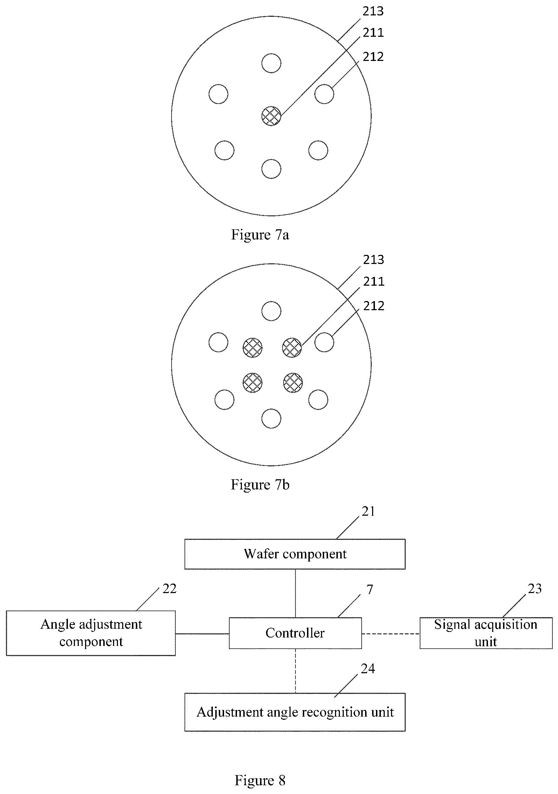

Figure 7a shows an optional arrangement of the plurality of wafers in the wafer component 21. There is one wafer arranged at the center position of the ultrasonic acquisition unit, and the periphery of the one wafer is surrounded by other wafers. The one wafer at the center position is referred to as a central wafer 211, and other wafers around the central wafer 211 are referred to as peripheral wafers 212.

In the case that the number of wafers is further increased, the wafers may also be arranged in an annular mode, the innermost annular layer is formed by the central wafers 211, and the outer annular layer is formed by the peripheral wafers 212. Wherein, Figure 7b shows another optional arrangement mode of the wafer component 21.

Based on this, in this embodiment, there is no limitation on the number of the central wafers 211 and the number of the peripheral wafers 212, and the corresponding arrangement may be specifically set according to actual situations.

In some optional embodiments, the controller further comprises an ultrasonic transceiving control module which is responsible for emission of an ultrasonic signal as well as receiving and processing of an ultrasonic echo signal by the wafers. As shown in Figure 7a or Figure 7b, each wafer emits an ultrasonic signal and receives an ultrasonic echo signal according to a certain timing sequence.

Specifically, when a wafer is one complete wafer, the ultrasonic transceiving control module may control the wafer to transmit an ultrasonic signal in the form of a pulse wave. When a wafer is composed of an ultrasonic transmitting half-wafer and an ultrasonic receiving half-wafer, the ultrasonic transceiving control module may control the wafer to transmit an ultrasonic signal in the form of a continuous wave.

In some optional embodiments, the angle adjustment component 22 is connected to the wafer component 21 to execute the target tracking by adjusting the angle of the wafer component 21.

Specifically, the angle adjustment component 22 may comprises adjustment members corresponding to wafers in the wafer component in a one-to-one manner to realize independent adjustment of the angle of each wafer; or, the respective wafers may also be fixed on a carrier, and the angle adjustment component 22 adjusts the angles of the wafers by adjusting the angle of the carrier. There are no restrictions on the specific adjustment modes herein, and specifically, the corresponding arrangement may be set according to actual needs.

Taking the case of the wafers being fixed on a carrier as an example, the angle adjustment component 22 may comprise a rotatable member and a connecting member connected to the rotatable member. Wherein, the rotatable member is fixed on the carrier to realize the rotation of the carrier, and the connecting member connects the rotatable member to the controller, and the rotation of the rotatable member is controlled by the rotation of the connecting member. The rotatable member may be a rotatable shaft or a universal rotatable member; the connecting member may be a transmission shaft, a connecting rod, etc. Or, the angle adjustment component 22 may be implemented in a manner of a rotatable mechanical arm, one end of the rotatable mechanical arm is connected to the carrier 213 in the wafer component 21, and the other end of the rotatable mechanical arm is connected to the controller.

As shown in Figure 7a or Figure 7b, the wafer component 21 further comprises a carrier 213, and each wafer is fixed on the carrier 213, and the angle adjustment component 22 is connected to the carrier 213 to adjust the angle of the carrier, so as to realize the simultaneous adjustment of the angles of all the wafers on the carrier 213. Because the plurality of wafers are arranged on the same carrier and the action of the carrier is used for driving all the wafers to act simultaneously, the adjustment time is reduced and the efficiency of the target tracking is improved.

As shown in Figure 8, the principle of the target tracking is as follows: the controller further comprises a signal acquisition unit, the signal acquisition unit is mainly used for ultrasonic demodulation, filter processing and energy calculation.

Specifically, after receiving an echo signal corresponding to each wafer, the signal acquisition unit obtains an ultrasonic Doppler frequency shift signal after going through an ultrasonic demodulation module and a filter processing module of the corresponding channel respectively. For example, each wafer corresponds to one signal processing channel, and the signal acquisition unit may distinguish the source of each echo signal by identifying the signal processing channel thereof.

The energy calculation module in the signal acquisition unit is responsible for calculating the energy of the ultrasonic Doppler frequency shift signal obtained by each wafer to obtain an energy signal of the corresponding channel, and performing fetal heart-rate validity check according to the energy signal. When the signal amplitude obtained in any one or more channels exceeds the minimum signal threshold, it is considered that a valid fetal heart-rate signal is inputted. When a valid fetal heart-rate signal is detected in any one or more channels of all the channels, the optimal position recognition and tracking function for the fetal heart-rate will be started.

In the case that the optimal position recognition and tracking function for the fetal heart-rate is started, the wafer channel having the strongest energy is judged in real time, and the locating of the channel with the strongest signal is completed. Otherwise, continuous search is performed until finding the wafer channel having a valid fetal heart-rate signal with the strongest energy.

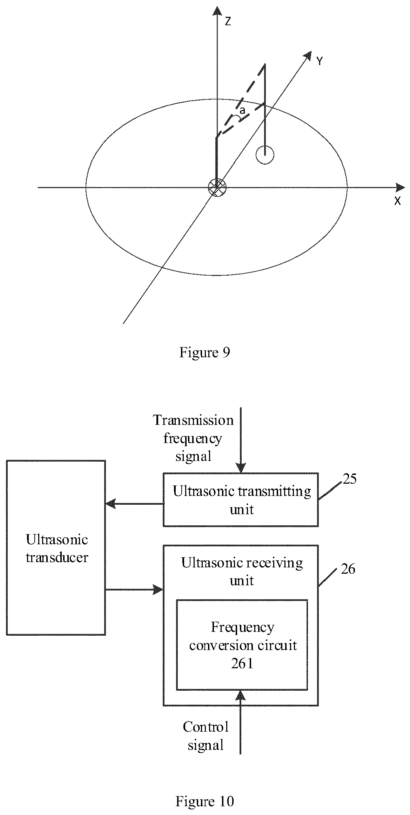

In some optional embodiments, the controller further comprises an adjustment angle recognition unit, the specific processing principle thereof is shown in Figure 9. Specifically, an XYZ three-axis coordinate system is established, with the center wafer position is used as an origin, the carrier in the wafer component is used as the XY plane, and the ultrasonic emission direction is used as the positive direction of the Z axis.

Firstly, determining the coordinate position of each wafer in the XYZ coordinate system described above according to the arrangement condition of the wafers in the wafer component 21, and the distance (Distance) between a peripheral wafer and the central wafer may be determined by using the coordinate position. That is, the spacing thereof is the distance between the coordinates of the central wafer and the coordinates of the peripheral wafer.

In the target tracking process, after the controller obtains the signal of each wafer, the controller completes the locating of the channel with the strongest signal in the manner described above to determine the wafer channel with the strongest energy. Specifically, when the channel with the strongest signal is a central wafer channel, it indicates that the ultrasonic acquisition unit is currently facing the fetal heart position, thus indicating that the angle of the wafer component does not need to be adjusted at this time, that is, the adjustment angle is set to 0. Otherwise, Figure 9 shows the schematic diagram of calculation of an adjustment angle $a$ when the signal strengths of the peripheral wafers are higher than that of the central wafer. Searching for the channel with the strongest signal among those of the peripheral wafers, and obtaining the coordinates of the wafer corresponding to the channel with the strongest signal, and calculating the energy difference $\Delta E$ between the wafer corresponding to the channel with the strongest signal and the central wafer channel. According to the inverse trigonometric function formula:

$$a = \arctan \frac{\Delta E}{Distance}$$

Wherein, Distance is the distance between the central wafer and the wafer corresponding to the channel with the strongest signal. In this way, the adjustment angle $a$ can be determined for the wafer component 21.

After determining the adjustment angle for the wafer component 21, the controller adjusts the wafer component 21 by using the angle adjustment component 22. Specifically:

When the adjustment angle $a$ is 0, it indicates that the wafer component does not need to be rotated;

When the adjustment angle is not 0, the rotation control for the wafer component 21 is performed by using the coordinate position of the wafer corresponding to the channel with the strongest signal in the XYZ coordinate system, so that the signal strength obtained by the central wafer gradually increases until the central wafer becomes the wafer with the strongest signal strength among all wafers.

For example, taking Figure 9 as an example, when the wafer corresponding to the channel with the strongest signal is in the first quadrant of the XY plane, the wafers in first quadrant are rotated towards the negative direction of the Z axis and the wafers in third quadrant are rotated towards the positive direction of the Z axis by a certain rotation control mode (for example, using a rotatable shaft having a rotation angle control function), until the channel with the strongest signal acquisition is switched to the center wafer channel. That is, the controller adjusts the rotation angle $a$ by controlling the angle adjustment component 22, so as to make the signal strength of the central wafer be the strongest.

In some optional embodiments, the ultrasonic acquisition unit further comprises a housing and a deformable filling material layer. Specifically, the deformable filling material layer is used for filling between the wafer component 21 and the housing, for example, using a flexible material, to ensure that no gap exists between the wafers and the housing, and meanwhile, the rotation of the wafer component is not hindered. Further, because the same deformable filling material layer is used for adjusting, adverse influence of refractive indices of multiple materials on signal transmission can be avoided, and the adjustment accuracy of the angle adjustment component is improved.

In some optional embodiments, the angle adjustment component may comprise at least one reflecting mirror component, wherein each of the at least one reflecting mirror component corresponds to at least one of the wafers, and is used for adjusting an ultrasonic emission direction of the at least one of the wafers. The reflecting mirror component may be formed by using a connecting member having angle rotation function and a reflecting mirror, one end of the connecting member is used for fixing the reflecting mirror and driving the reflecting mirror to rotate, and the other end of the connecting member is connected with the controller.

Specifically, only one reflecting mirror component or a plurality of reflecting mirror components may be arranged in the ultrasonic acquisition unit. For example, two or more reflecting mirror components may be arranged, etc. The specific number of the reflecting mirror components may be correspondingly arranged according to actual needs, which is not limited herein.

As described above, the purpose of adjusting the wafer component 21 is to make the central wafers in the wafer component 21 directly face the target. Therefore, when the reflection directions of the central wafers are not aligned with the target, the ultrasonic signals emitted by the wafers are reflected to the detection target by adding the reflecting mirror component. Specifically, the action of the reflecting mirror component is controlled by the angle adjustment component 22 to realize changing the ultrasonic signal emission angle and achieving the tracking of the fetal heart-rate signal.

Wherein, whether the reflection directions of the central wafers are directly facing the target may be determined by comparing the signal strengths of the respective wafers. If the signal strength of the central wafers is not the strongest in all the wafers, it may be determined that the wafer component needs to be adjusted by using the reflecting mirror component. The specific adjustment manner is similar to the angle adjustment manner described above, which is determined by the position relationship between the wafer with the strongest signal and the central wafers and the difference value of signal strength.

Because the target tracking is realized by changing the ultrasonic emission direction of the wafers, direct contact between the angle adjustment component and the wafer component is avoided, and mutual influence between their respective actions can be avoided.

Further, according to the function of the software program therein, the controller may further comprise a signal acquisition unit 23 and a rotation angle recognition unit 24. The signal acquisition unit 23 is used for receiving ultrasonic echo signals corresponding to the respective wafers, and processing the ultrasonic echo signals, and determining the signal strength corresponding to each wafer; then, the rotation angle recognition unit 24 is used for determining the size of the adjustment angle based on the corresponding signal strength of each wafer.

The ultrasonic acquisition unit in the embodiments of the present disclosure is further used for realizing synchronous adjustment of the transmitting frequency of an ultrasonic transmitting unit and the receiving frequency of an ultrasonic receiving unit, thereby realizing that the frequency response feature of the ultrasonic receiving unit is matched with the transmitting frequency, and obtaining the optimal receiving signal-to-noise ratio.

One purpose of the ultrasonic acquisition unit is to ensure the synchronous adjustment of the ultrasonic transmitting frequency and the ultrasonic receiving frequency, so as to ensure the consistency between the ultrasonic transmitting frequency and the ultrasonic receiving frequency. In the existing schemes, the controller is generally used for generating a transmission frequency signal, for example, a frequency pulse. Based on this, it is equivalent to determining the receiving frequency of the ultrasonic receiving unit. If the receiving frequency of the ultrasonic receiving unit is adjusted based on the resonance principle, the resonant principle is to form a resonant circuit based on an inductor together with a capacitor or a resistor, the resonant frequency can be adjusted by changing the value of the inductor's inductance, the capacitor's capacitance or the resistor's resistance. Therefore, the controller may determine the control signal by using the composition of the resonant circuit to calculate the adjusted inductance, capacitance, or resistance value after determining the receiving frequency, so as to switch the circuit parameters in the frequency conversion circuit.

For example, three resonant frequencies may be provided by the frequency conversion circuit, and when the circuit is connected, the three resonant frequencies are connected to the corresponding pins of the controller. Accordingly, three resonant frequencies are recorded in the controller, and after the receiving frequency is determined, the controller may control one corresponding pin to output a corresponding control signal to select one resonant frequency in the frequency conversion circuit.

Of course, the way of the controller generating a control signal is not limited to the foregoing, but may be implemented in other manners, which is not limited herein, and it is only necessary to ensure that the control signal corresponds to the transmission frequency signal.

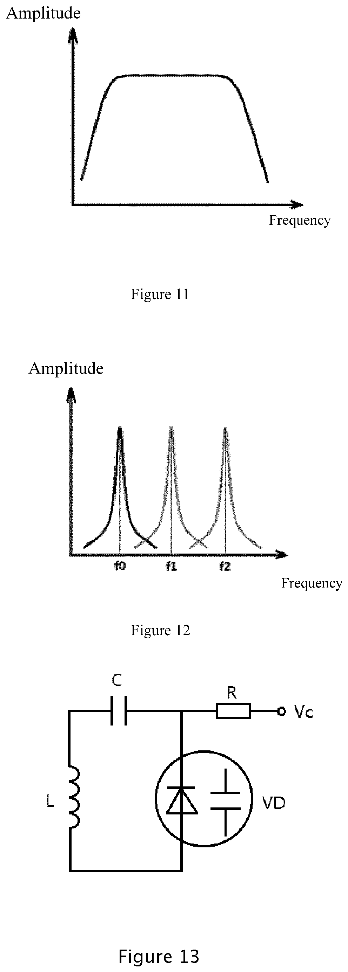

Specifically, the ultrasonic receiving unit and the ultrasonic transmitting unit conduct frequency conversion synchronously. Because the frequency conversion circuit is introduced into the ultrasonic receiving unit, the frequency response features of the ultrasonic receiving unit can obtain a good quality factor at each working frequency point. Figure 11 shows the receiving bandwidth in prior art, and Figure 12 shows the receiving bandwidth in this embodiment, it can be known from the comparison thereof that the bandwidth in this embodiment is much narrower than that in the prior art, it has better frequency selection features for useful ultrasonic echo signals, and more out-of-band noise can be filtered out, so as to realize the purpose of achieving the optimal signal-to-noise ratio of the ultrasonic receiving unit under each working frequency point, and thereby the ultrasonic emission energy is reduced.

As shown in Figure 10, the ultrasonic acquisition unit comprises an ultrasonic transmitting unit 25 and an ultrasonic receiving unit 26, and the ultrasonic transmitting unit comprises a frequency conversion circuit 261. Wherein, the ultrasonic transmitting unit 25 is used for driving an ultrasonic transducer based on a transmitting frequency signal to generate an ultrasonic wave; the ultrasonic receiving unit 26 is used for receiving the ultrasonic echo to obtain a detection result. The ultrasonic transducer is an acoustoelectric and electroacoustic conversion sensor, when the ultrasonic transmitting unit outputs an electrical signal to drive the ultrasonic transducer, the ultrasonic transducer converts the electrical signal into an acoustic wave signal. When the acoustic wave signal is propagating in the detection object's tissues, a portion of the acoustic energy thereof is reflected back to the ultrasonic transducer, and the ultrasonic transducer inversely converts this portion of the acoustic energy into an electrical signal and transmits the electrical signal to the ultrasonic receiving unit 26.

Specifically, the ultrasonic transmitting unit 25 is connected to the controller, and the controller is used for generating a transmission frequency signal for the ultrasonic transmitting unit 25. The ultrasonic transmitting unit 25 receives the transmission frequency signal, and drives the ultrasonic transducer after processing the transmission frequency signal. Since the transmission frequency signal generated by the controller is a digital signal, it cannot directly drive the ultrasonic transducer, therefore, the ultrasonic transmitting unit 25 is required to output an analog signal after performing power amplification and other processing on the digital signal, and drive the ultrasonic transducer by using the analog signal. Wherein, the controller in the ultrasonic acquisition unit may be the controller in the fetal Doppler, or it may be a separate controller, etc.

The ultrasonic receiving unit 26 has a frequency conversion circuit connected to the controller, the controller is further used for generating a control signal corresponding to the transmission frequency signal, and the control signal is for adjusting the circuit parameter of the frequency conversion circuit to adjust the receiving frequency of the ultrasonic receiving unit 26. Specifically, the frequency conversion circuit may change the receiving frequency of the ultrasonic receiving unit based on the resonance principle, that is, the resonant frequency of the frequency conversion circuit is changed by adjusting the circuit parameter of the frequency conversion circuit, so as to adjust the receiving frequency. Alternatively, the frequency conversion circuit may change the receiving frequency based on the active filtering principle, that is, the filtering bandwidth of the frequency conversion circuit is changed by adjusting the circuit parameter of the frequency conversion circuit, so as to adjust the receiving frequency.

By providing a frequency conversion circuit in the ultrasonic receiving unit, the frequency conversion circuit and the ultrasonic transmitting unit are controlled by the controller at the same time, when the transmission frequency of the ultrasonic transmitting unit is adjusted, the circuit parameter of the frequency conversion circuit is adjusted at the same time to realize the synchronous adjustment for the receiving frequency, so that the frequency response features of the ultrasonic receiving unit can obtain a good quality factor at each working frequency point, so that the frequency selection features for the useful ultrasonic echo signal is better, and more out-of-band noise can be filtered out, and the purpose of achieving the optimal signal-to-noise ratio of the ultrasonic receiving unit under each working frequency point is realized.

In some optional embodiments, when the frequency conversion circuit changes the receiving frequency of the ultrasonic receiving unit based on the resonance principle, the frequency conversion circuit may be referred to as a tuning circuit, and correspondingly, the control signal outputted by the controller is used for adjusting the resonant frequency of the tuning circuit. The resonant frequency of the tuning circuit may be adjusted by adjusting the value of a capacitor's capacitance, an inductor's inductance or a resistor's resistance in the tuning circuit. For example, the tuning circuit comprises a capacitor and an inductor, and while the inductor's inductance remains unchanged, the value of the capacitor's capacitance is changed to adjust the resonant frequency.

Specifically, the frequency conversion circuit is an LC tuning circuit, comprising an inductor and an adjustable capacitor module. The adjustable capacitor module is connected in parallel with the inductor, and the adjustable capacitor module is connected to the controller. Wherein, the adjustable capacitor module changes the capacitance value in the frequency conversion circuit under the action of the control signal of the controller to realize the adjustment of the resonant frequency.

In some optional embodiments, as shown in Figure 13, the control signal is a voltage signal Vc, and the adjustable capacitor module comprises a varactor VD. The control signal is a reverse voltage applied to the varactor VD. With the change of the reverse voltage, the capacitance value of the varactor VD can be changed. The varactor VD with a variable capacitance value can cooperate with the inductor L to form a variable frequency conversion circuit.

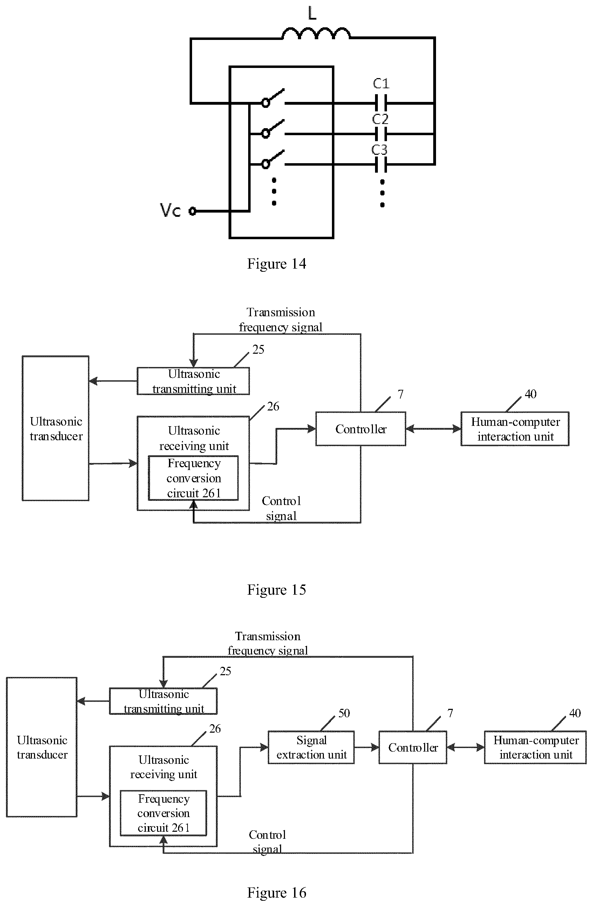

In some optional embodiments, the adjustable capacitor module comprises at least two capacitor branch circuits connected in parallel, and the controller is connected to the at least two capacitor branch circuits, each of the capacitor branch circuits comprises a first controllable switch and a first capacitor connected in series, the capacitance value of the first capacitor in each of the capacitor branch circuits is different or the same, there is no restriction on the specific value of the capacitance herein, which may be set according to actual needs. When the capacitance value of the first capacitor in each of the capacitor branch circuits are the same, the number of the capacitor branch circuits that are turned on at the same time may be controlled, and the capacitance value of the circuit may also be adjusted. For example, as shown in Figure 14, it comprises at least three capacitor branch circuits, and the capacitance values of the corresponding first capacitors are C1, C2 and C3 respectively. The capacitance values of these first capacitors are different, and thus different resonant frequencies can be obtained by combining different capacitor branch circuits with the inductor L, and thereby different receiving frequencies can be obtained. For example, as for the controller, for different transmission frequency signals, the controller outputs different voltage values, and the conditions for turning on the first controllable switches in each of the capacitor branch circuits are different, different voltage values may be used for turning on different first controllable switches, and thereby different capacitor branch circuits are selectively gated.

In some optional embodiments, when the frequency conversion circuit changes the receiving frequency based on the active filtering principle, the frequency conversion circuit is an active filter amplification circuit, and the control signal is used for adjusting a bandwidth and a center frequency of the active filter amplification circuit. The active filter amplification circuit is used for performing band-pass filtering on the received signal, and by changing the bandwidth of the band-pass filtering, the receiving frequency of the ultrasonic receiving unit can be adjusted.

Specifically, the active filter amplification circuit comprises at least two bandwidth branch circuits connected in parallel, the controller is connected to the at least two bandwidth branch circuits, each of the bandwidth branch circuits comprises a second controllable switch and a target element connected in series, and the target element comprises a second capacitor and/or a resistor.

Wherein, the first controllable switch and the second controllable switch may be an analog switch, an electric relay or a triode, etc., which is not limited herein. It should be noted that the control signal outputted by the controller may be one or more, that is, at least two capacitor branch circuits or bandwidth branch circuits are controlled by using one same control signal; alternatively, a plurality of control signals are used for controlling at least two capacitor branch circuits or bandwidth branch circuits respectively, that is, the control signals correspond to the capacitor branch circuits or bandwidth branch circuits in a one-to-one manner.

The ultrasonic receiving unit 26 is configured to receive a weak ultrasonic echo signal of the ultrasonic transducer, and perform frequency selection amplification on the echo signal. As described above, frequency selection is performed by using the frequency conversion circuit, such as a tuning circuit composed of an inductor and a capacitor, or an active filter amplification circuit composed of an operational amplifier, a resistor and a capacitor. The circuit parameter adjustment of the frequency conversion circuit, comprising adjustment of inductance, capacitance, resistance, etc., results in the change of the frequency selection feature, which is matched with the current ultrasonic transmission frequency, so that the useful frequency component in the echo signal would be amplified by the ultrasonic receiving unit, while the useless frequency component would be filtered out by the ultrasonic receiving unit, so as to obtain the echo signal with lower noise.

In some optional embodiments, the ultrasonic detection circuit further comprises a controller. That is, a separate controller is provided in the ultrasonic detection circuit to control the transmitting and receiving frequencies to make the controller independent of other controllers, and ensure the reliability of control.

In some optional embodiments, as shown in Figure 15, the ultrasonic acquisition unit further comprises a human-computer interaction unit 40, the human-computer interaction unit 40 is connected to the controller to display a detection result of the ultrasonic detection circuit. Specifically, the human-computer interaction unit may be a button, a display member, etc.

In some optional embodiments, the human-computer interaction unit comprises a first adjustment member for adjusting the transmission frequency of the ultrasonic transmitting unit. For example, the first adjustment member is arranged on the housing, and the detection object can adjust the transmission frequency by operating the first adjustment member.

Wherein, the detection results comprise fetal heart-rate audio. The human-computer interaction unit further comprises an audio output member and a second adjustment member, the audio output member is used for playing the fetal heart-rate audio; the second adjustment member is connected to the audio output member, and the second adjustment member is used for adjusting the volume of the fetal heart-rate audio.

As shown in Figure 16, the ultrasonic acquisition unit further comprises a signal extraction unit 50 connected to the output of the ultrasonic receiving unit 26 and configured to perform demodulation, sampling-and-holding, filtering, amplification, etc. on the signal outputted by the ultrasonic receiving unit 26, and shift the spectrum of the high-frequency signal to the audio signal.

As shown in Figure 16, the detection object adjusts the transmission frequency through the human-computer interaction unit 40, and accordingly, the adjusted transmission frequency is outputted to the controller. The controller generates a transmission frequency signal and a control signal based on the adjusted transmission frequency, and sends the transmission frequency signal to the ultrasonic transmitting unit 25, and sends the control signal to the frequency conversion circuit of the ultrasonic receiving unit 26. The ultrasonic transmitting unit 25 processes the transmission frequency signal and then drives the ultrasonic transducer to transmit an ultrasonic wave, and the ultrasonic transducer further receives the ultrasonic echo and sends the ultrasonic echo to the ultrasonic receiving unit 26. The frequency conversion circuit 261 adjusts the receiving frequency of the ultrasonic receiving unit 26 under the action of the control signal. Correspondingly, the ultrasonic receiving unit 26 performs frequency selection and other processing on the ultrasonic echo signal based on the receiving frequency, and sends the processed signal to the signal extraction unit 50. The signal extraction unit 50 is used to perform demodulation, sampling-and-holding, filtering, amplification, etc. on the signal outputted by the ultrasonic receiving unit 26, and send the detection result to the human-computer interaction unit 40 through the controller to output the detection result.

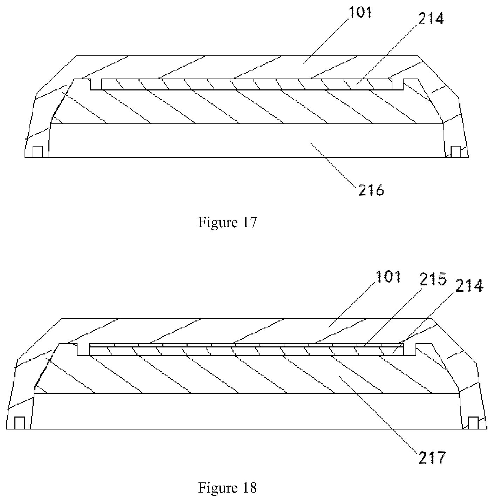

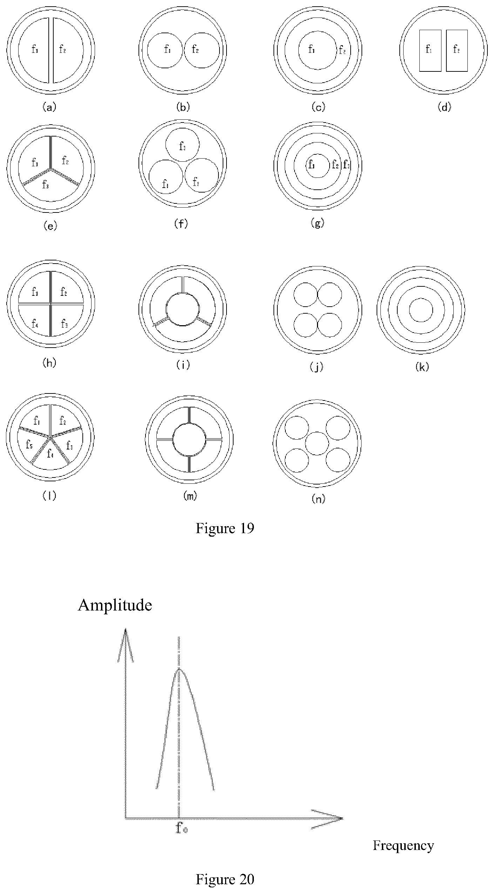

As shown in Figure 17, an existing ultrasonic acquisition unit generally comprises an end face housing 101, a wafer layer 214 and a casting adhesive layer 216. The end face housing and the columnar housing are sealedly bonded by an epoxy resin adhesive, and the wafer layer is integrally bonded to the inner surface of the end face housing by a special glue. The positive and negative electrodes of the wafer are respectively led out through leads. A certain amount of casting adhesive encapsulates the back face of the wafer on the end face housing, which can play a role in protecting the wafer layer. However, as shown in Figure 20, this kind of ultrasonic acquisition unit adopts fixed frequency output and is unable to adapt to pregnant women with different gestational weeks and different obesity levels, so it is difficult for this kind of ultrasonic acquisition unit to obtain high-quality signals, which ultimately adversely affects the actual clinical detection effect. The existing ultrasonic acquisition unit basically works at a single frequency, if different frequencies are needed for different detection objects, it needs to be realized by configuring ultrasonic acquisition units with different frequencies, that is, by using two or more ultrasonic acquisition units with different frequencies, or by embedding multiple wafers inside the ultrasonic acquisition unit, this will inevitably lead to the complexity and inconvenience of the detection procedure and increase the detection cost.

In view of this, the ultrasonic acquisition unit in this embodiment is provided with a variable-frequency wafer module, which is arranged inside the end face housing of the housing to adjust a working frequency of the ultrasonic acquisition unit. Due to the existence of the variable-frequency wafer module, the fetal Doppler can realize detection by only one ultrasonic acquisition unit when treating pregnant women with different gestational weeks and obesity levels, a user can select the appropriate working frequency of the ultrasonic acquisition unit to acquire the optional signal quality and improve the detection result, and the difficulty of operation and detection and the cost of medical equipment are reduced, making fetal heart-rate detection more efficient and convenient.

The variable-frequency wafer module may be at least one medium layer arranged between the wafer layer of the ultrasonic acquisition unit and the end face housing; or may comprise at least two wafers, wherein the inherent vibration frequencies of the respective wafers are not all the same.

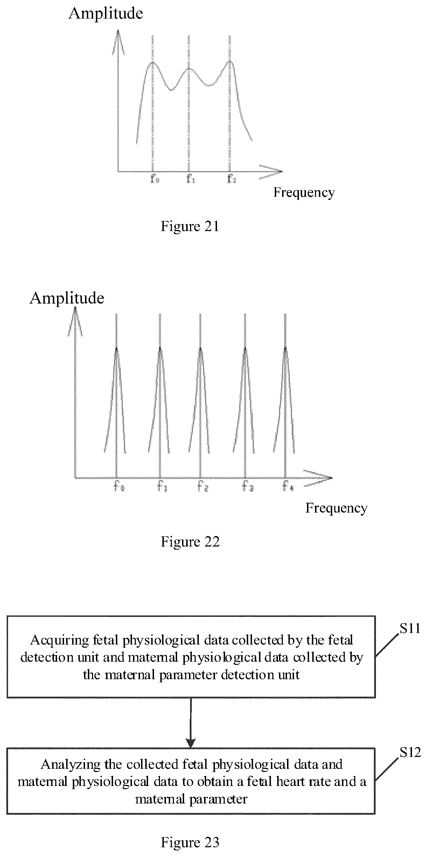

Wherein, as shown in Figure 18, a medium layer 215 is added between the wafer layer 214 and the end face housing 101, the setting of the medium layer 215 is used for changing an inherent vibration frequency of the wafer layer 214, thereby facilitating the ultrasonic waves in more frequency bands being received by the detection object. On the other hand, the arrangement of the medium layer improves the impedance features of the propagation among the wafer layer, the end face housing and the detection object, so that the ultrasonic energy can be propagated more effectively, thereby the sensitivity of the ultrasonic acquisition unit is further improved. As shown in Figure 21, after adding the medium layer, the ultrasonic acquisition unit can output a plurality of working frequencies, not limited to one working frequency.

The thickness of the medium layer 215 is one quarter of the wavelength, and the material of the medium layer may be glass, graphite and other solid materials, or may be epoxy resin, plastic, or it is made by adding tungsten powder or aluminum oxide powder into epoxy resin according to a certain proportion, stirring and curing. For the medium layer, it may be a single layer or multiple layers, the number of the medium layers is mainly determined by material characteristics and thickness dimensions.

In some optional embodiments, as shown in Figure 18, the ultrasonic acquisition unit further comprises a backing layer 217, the backing layer 217 is arranged on one side of the variable-frequency wafer module away from the end face housing 101. Specifically, because, in the process of wafer vibration, in addition to transmitting an ultrasonic wave to the front end, it would also transmit ultrasonic wave to the rear end of the wafer and propagate some energy, thus the ultrasonic wave generated at the rear end of the wafer would be reflected to the front end of the wafer by a certain extent. In an actual design process, it is desired that the ultrasonic wave at the rear face of the wafer is not reflected to the front end face or is reflected thereto as less as possible. Therefore, a backing layer is arranged on the back face of the wafer, the acoustic impedance of the material of the backing layer is far smaller or far larger than the acoustic impedance of the wafer. Based on this, in this embodiment, a backing with a high attenuation coefficient is selected, so that the ultrasonic waves of different frequency bands emitted from the rear end face of the wafer can be absorbed, and enter into the backing, so that these ultrasonic waves are prevented from re-returning back to the front end of the wafer after being reflected, because the reflected ultrasonic waves would adversely influence the fetal heart-rate signal and the detection effect.

Further, the ultrasonic acquisition unit comprises at least two wafers, for example, 2-5 wafers, each of the wafers has its own inherent vibration frequency. The working frequency range of the acquisition unit covers 1.0 MHZ ~ 5 MHZ. As shown in Figures 19a - 19n, the shape of the wafers includes at least one of a circular shape, a rectangular shape, an elliptical shape and a sector shape. For the ultrasonic acquisition unit, the shape of the wafers comprised in the ultrasonic acquisition unit may be one of the shapes described above, or a geometric shape formed by combining at least two of the shapes described above, etc. For example, if the ultrasonic acquisition unit comprises wafers with five inherent vibration frequencies, the ultrasonic acquisition unit can work at five working frequencies as shown in Figure 22. Of course, Figure 21 and Figure 22 are merely examples. The working frequency of the ultrasonic acquisition unit may be set according to actual needs.

The arrangement manner of the at least two wafers described above comprises a dot-matrix arrangement, a ring-matrix arrangement, or an arrangement that equally divides the ultrasonic acquisition surface of the ultrasonic acquisition unit. In specific use, the detection object may select the wafer with an appropriate frequency band according to the features of the detection object to operate. Because the inherent vibration frequencies of the respective wafers are not all the same, it is suitable for pregnant women with different gestational weeks and obesity levels that the appropriate working frequency of the fetal heart-rate ultrasonic acquisition unit can be selected for different detection objects to obtain the optimal signal quality and improve the measurement result.

According to the embodiments of the present disclosure, an embodiment of a detection method of the fetal Doppler is provided. It should be noted that the steps shown in the flowcharts of the drawings may be executed in a computer system such as one having a set of computer-executable instructions stored therein, and although the logical sequence is shown in the flowcharts, in some cases, the steps shown or described may be executed in a sequence different from herein.

In this embodiment, a detection method of fetal Doppler is provided, which may be applied to the above-mentioned controller, etc. Figure 23 is a flowchart of the detection method of the fetal Doppler according to this embodiment of the present disclosure, as shown in Figure 23, the process comprises the following steps:

S11, Acquiring fetal physiological data collected by the fetal detection unit and maternal physiological data collected by the maternal parameter detection unit.

S12, Analyzing the collected fetal physiological data and maternal physiological data to obtain a fetal heart rate and a maternal parameter.

Specifically, the structural details of the fetal detection unit and the maternal parameter detection unit in the fetal Doppler can refer to the description above and will not be repeatedly described herein. For the controller, after acquiring the fetal physiological data and the maternal physiological data, the acquired fetal physiological data and maternal physiological data are analyzed by the controller to obtain a fetal heart rate and a maternal parameter. The specific analysis process can refer to the description above and will not be repeatedly described herein.

In some optional embodiments, after acquiring the fetal heart rate and maternal heart rate, the controller is further configured to perform preset processing on the acquired fetal heart rate and maternal parameter. After the fetal heart rate and maternal heart rate are acquired by the fetal Doppler, the preset processing performed by the controller on the fetal heart rate and maternal parameter may be in various processing manners such as display, processing, analysis, etc., which is not specifically limited herein.

In some optional embodiments, when the controller performs preset processing on the fetal heart rate and maternal parameter, the fetal heart rate may be displayed, for example, the fetal heart rates corresponding to different time points are directly displayed; for another example, a fetal heart-rate curve is generated by using the fetal heart rates corresponding to different time points, and the fetal heart-rate curve is displayed.

In some optional embodiments, when the controller performs preset processing on the fetal heart rate and the maternal parameter, the maternal parameter may be displayed, for example, the maternal parameters corresponding to different time points are directly displayed; for another example, a maternal heart-rate curve is generated by using maternal heart rates corresponding to different time points, and the maternal heart-rate curve is displayed.

In some optional embodiments, when the controller performs preset processing on the fetal heart rate and the maternal parameter, the controller may determine status information about the fetal heart rate and/or the maternal parameter, and display the status information. The state information may be a result after the fetal heart rate and the maternal heart rate are analyzed and processed, for example, the state information may comprise at least one of the following: quality information of the fetal heart rate and quality information of the maternal parameter. The quality information is used for indicating the accuracy, reliability, etc. of the heart rate, and the manner of obtaining the quality information is not specifically limited herein.

The maternal parameter comprises a maternal heart rate. In order to compare and verify the fetal heart rate and the maternal heart rate, overlap between the fetal heart rate and the maternal heart rate is detected, so that an alarm prompt is realized, an overlapping interval between the fetal heart rate and the maternal heart rate may also be detected, and subsequent alarm processing is performed. Specifically: firstly, the fetal Doppler uses the fetal detection unit and the maternal parameter detection unit to correspondingly collect fetal physiological data and maternal physiological data respectively. Then, the controller analyzes the fetal physiological data and maternal physiological data respectively to obtain the fetal heart rate and the maternal heart rate. Finally, the controller is further used for detecting an overlapping interval between the fetal heart rate and the maternal heart rate, and if the overlapping interval is detected, it will send a prompt and/or mark the overlapping interval on a displayed heart rate curve.

The overlapping interval between the fetal heart rate and the maternal heart rate may be a portion where the fetal heart rate and the maternal heart rate are identical to each other or approximate to each other, which is detected after comparing and verifying the fetal heart rate and the maternal heart rate. The overlapping interval between the fetal heart rate and the maternal heart rate indicates the presence of the possibility of taking a maternal heart rate as a fetal heart rate, or indicates that the heart rate value may be incorrect. When preset processing is performed on the fetal heart rate and the maternal heart rate, the overlapping interval of the fetal heart rate and the maternal heart rate may be detected, so that an alarm may be implemented by prompt and in other manners when the fetal heart rate and the maternal heart rate are determined to overlap each other.

In some optional embodiments, when the controller detects the overlapping interval between the fetal heart rate and the maternal heart rate, it may acquire a first number of heart rate differences respectively corresponding to different moments within a first time period, wherein, the heart rate difference corresponding to each moment is a difference feature value between the fetal heart rate and the maternal heart rate at the moment; count a second number of the heart rate differences satisfying a first condition within the first time period; if the second number satisfies a second condition and the heart rate differences within a second time period in the first time period all satisfies the first condition, determine that the first time period is an overlapping interval between the fetal heart rate and the maternal heart rate. The lengths of the first time period and the second time period may be custom set, it is only required that the second time period is less than or equal to the first time period. The heart rate difference is a difference feature value between the fetal heart rate and the maternal heart rate, which may be a difference or a standard deviation between the fetal heart rate and the maternal heart rate, for example, a standard deviation sequence between the fetal heart rate and the maternal heart rate is calculated. The first condition may be that the heart rate difference is equal to a preset heart rate difference value, etc.; it may be that the heart rate difference is less than a preset threshold, and it may also be other conditions indicating that the fetal heart rate and the maternal heart rate approximate to each other or overlap each other. The second condition may be that the proportion of the second number in the first number is greater than a preset proportion, and may be that the second number is greater than a preset number value, etc.

If the overlapping interval is detected by the controller, a prompt is sent and/or the overlapping interval is marked on a displayed heart rate curve. If the overlapping interval between the fetal heart rate and the maternal heart rate is detected, a prompt may be sent. The prompt way may be any one of the prompt implementation ways, and there is no specific restriction herein. For example, the prompt is done by at least one of the following: displaying prompt information, playing a prompt sound, turning on a prompt lamp, etc. The fetal Doppler may comprise a display screen, a speaker, a prompt lamp, etc., which are connected to the controller, wherein, the display screen may be used for displaying prompt information, the speaker may be used for playing the prompt sound, and the prompt lamp may prompt a detection object by its light, so that the controller detects the overlapping interval and controls the display screen, the speaker, the prompt lamp or other devices connected with the controller, and thereby an alarm is done by displaying of the display screen, a sound, light, etc.

If the overlapping interval between the fetal heart rate and the maternal heart rate is detected by the controller, the overlapping interval may be marked on the displayed heart rate curve. The heart rate curve comprises at least one of the following: a fetal heart rate curve generated from the fetal heart rate and a maternal heart rate curve generated from the maternal heart rate. The marking may be done by any one of the marking implementation ways, without specific restriction herein. In an embodiment, after generating a fetal heart rate curve by using fetal heart rates corresponding to different time points and generating the maternal heart rate curve by using maternal heart rates corresponding to different time points, if an overlapping interval between the fetal heart rate and the maternal heart rate is detected, the controller may be configured to replace a color or a line type of a line segment corresponding to the overlap interval in the heart rate curve, or add a preset mark on a line segment corresponding to the overlap interval in the heart rate curve. Of course, if an overlapping interval between fetal heart rate and the maternal heart rate is detected, the alarm may also be done in other ways.

By means of the above prompt, marking or other alarm ways, when heart rate overlap exists between the fetal heart rate and the maternal heart rate, an alarm can be done in time, so as to prompt a detection object of the fetal Doppler to adjust the position of the fetal Doppler, confirm the signal source, re-collect the fetal physiological data and the maternal physiological data, so as to obtain the fetal heart rate and the maternal heart rate after analyzing, thereby preventing the situation of taking the maternal heart rate measured by the fetal Doppler mistakenly as the fetal heart rate and introducing unnecessary clinical intervention.

In order to perform preset processing on the fetal heart rate and the maternal heart rate, the fetal Doppler may further comprise at least one functional component connected to the controller, and the functional component comprises at least one of a display screen, a sound playing component and a prompt lamp. The display screen is configured to display in response to an instruction of the controller. When the display screen displays in response to the instruction of the controller, it includes, but not limited to, displaying the fetal heart rate, displaying the maternal heart rate, displaying status information, displaying the fetal heart rate curve, displaying the maternal heart rate curve, displaying a heart rate curve marked with the overlapping interval, displaying the prompt information, etc. The fetal Doppler may further comprise a sound playing component such as a speaker which is connected to the controller and used for playing a prompt sound, or other alarm processing components such as a prompt lamp used for performing alarm processing, which is not specifically limited herein.

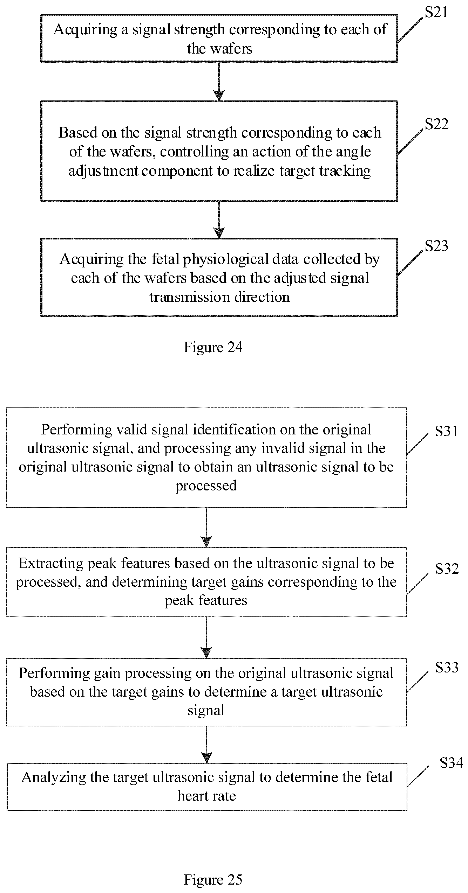

Because, in the fetal heart-rate monitoring process, the fetus may squirm, especially during the birth process, the fetus will move downwards continuously along with the birth process, and in order to obtain a continuous and stable fetal heart-rate signal, a detection object needs to pay attention to the fetal heart-rate signal quality in real time in the fetal monitoring process, and constantly adjust the position of the ultrasonic acquisition unit according to the fetal heart signal quality. In view of this, in order to solve the problem that a valid fetal heart-rate signal is unable to be obtained due to that the fetal heart deviates from the ultrasonic coverage of the ultrasonic acquisition unit, in this embodiment, the specific structure of the ultrasonic acquisition unit is shown in Figures 6- 7, and the ultrasonic acquisition unit comprises a plurality of wafers and an angle adjustment component. By automatically adjusting the rotation angle of each wafer, the signal strengths of the central wafers are made the strongest. Based on this, as shown in Figure 24, the above step of acquiring fetal physiological data collected by the fetal detection unit comprises the following steps:

S21, Acquiring the signal strength corresponding to each of the wafers.

The controller controls the respective wafers to emit ultrasonic signals in a certain time sequence and receives ultrasonic echo signals corresponding to the respective wafers. As mentioned above, in the controller, different channels are used for distinguishing the respective wafers, so the signal strength corresponding to each individual wafer can be determined by performing individual processing corresponding to each processing channel.

The specific processing procedure may refer to the corresponding processing described in the embodiments illustrated above, and will not be repeatedly described herein.

S22, Based on the signal strength corresponding to each of the wafers, controlling an action of the angle adjustment component to realize target tracking.

Wherein, the angle adjustment component is configured to adjust a signal transmission way between each of the wafers and a target body.

After determining the signal strength of each of the wafers, the controller can determine the position of the wafer with the strongest signal strength, and realize target tracking by controlling the action of the angle adjustment component, so as to ensure that the wafer corresponding to the target signal output has the strongest signal strength.

For example, subsequently, the signal corresponding to a wafer A is used for calculating the fetal heart rate, then, the signal strength corresponding to the wafer A is made the strongest by the action of the angle adjustment component, so as to ensure the reliability of the subsequent fetal heart rate calculation. As shown in Figure 7a or Figure 7b, if the wafer A is a central wafer, then the action of the angle adjustment component makes the signal strength corresponding to the central wafer be the strongest among all wafers. The action of the angle adjustment component is controlled based on the signal strength corresponding to each of the wafers, that is, the wafer component can always follow the target by means of target locating so as to obtain an accurate fetal heart-rate signal.

S23, Acquiring the fetal physiological data collected by each of the wafers based on the adjusted signal transmission direction.

After the signal transmission directions are adjusted for the wafers, each of the wafers collects the fetal physiological data based on the adjusted signal transmission direction, and sends the collected fetal physiological data to the controller, so that the controller performs analysis and calculation of the fetal heart rate.

Further, as described above, the plurality of wafers are divided into central wafers and peripheral wafers, and the peripheral wafers are arranged around a periphery of the central wafers. Based on this, the above step S22 may comprise the following steps:

S221, Comparing the signal strength of the plurality of wafers, and judging whether the wafer having the strongest signal strength is a central wafer.

If the wafer having the strongest signal strength is a peripheral wafer, step S222 is executed; otherwise, determining that the angle adjustment component does not need to perform adjustment.

S222, Calculating a signal strength difference value between the signal strength of the central wafer and the strongest signal strength;

After determining the wafer having the strongest signal strength (referred to as a target wafer), the controller can determine the signal strength difference value between the central wafer and the target wafer.

S223, Based on the signal strength difference value and the position of the wafer having the strongest signal strength, determining an adjustment angle and direction of the angle adjustment component.

As mentioned above, since the coordinate position of each wafer in the XY plane is determined, the position relationship between the central wafer and the target wafer is also determined. The controller can determine the adjustment angle and direction of the angle adjustment component based on the calculation principle shown in Figure 9, the purpose of the adjustment is to make the signal strength of the central wafer be the strongest among all wafers. The adjustment angle and direction of the angle adjustment component are determined by using the signal strengths of the central wafer and the peripheral wafers, so that the central wafer always becomes the wafer having the strongest signal strength among all the wafers, thereby ensuring the detection effect for the target.

In some optional embodiments, before the signal strength comparison is performed, the controller may firstly screen the received ultrasonic echo signals of the respective wafers to obtain valid signals; then the signal strengths of the valid signals are compared. The specific screening process may be as follows: comparing the ultrasonic echo signal of each wafer with a preset value, and if the intensity of the ultrasonic echo signal is greater than the preset value, it indicates that the ultrasonic echo signal is a valid signal; otherwise, the ultrasonic echo signal is considered as an invalid signal, and will be ignored in the subsequent comparison process of the signal strengths.

It is not easy to find a tiny fetal heart hidden in the uterus from the abdomen of a pregnant woman by using the fetal Doppler, and the fewer the pregnancy weeks of the pregnant woman are, the smaller the fetal heart is, and the weaker the Doppler frequency shift signal reflected from the fetal heart is, therefore, a very large gain parameter needs to be adopted to amplify the Doppler frequency shift signal in order to hear a fetal heart-rate sound and perform the fetal heart rate calculation. However, in the gain amplification process, not only useful signals will be amplified, but also noise signals will be amplified. If all the acquired signals are directly amplified uniformly, the fetal heart-rate beat signal will be unclear, and white noise would be amplified if the ultrasonic acquisition unit does not obtain a valid signal; when the fetus has intermittent limb movements and burps, due to the relatively fast speed of such movements, a relatively large Doppler frequency shift signal will be intermittently generated, and after a large gain amplification, the signal will be excessively amplified to be saturated, with a broken sound generated, which makes people feel very harsh and adversely affects the actual use. In view of this, in the embodiments of the present disclosure, when the collected fetal physiological data is analyzed to obtain the fetal heart rate, the fetal physiological data is first de-noised, and then the de-noised data is used for calculating the fetal heart rate. For the convenience of the following description, the collected fetal physiological data is referred to as an original ultrasonic signal, and the de-noised data is referred to as a target ultrasonic signal. Specifically, as shown in Figure 25, the above step of analyzing the collected fetal physiological data to obtain a fetal heart rate comprises the following steps:

S31, Performing valid signal identification on the original ultrasonic signal, and processing any invalid signal in the original ultrasonic signal to obtain an ultrasonic signal to be processed.

After the ultrasonic acquisition unit is started, the ultrasonic acquisition unit transmits a fixed frequency ultrasonic signal, when the ultrasonic signal touches a motion interface, the ultrasonic signal is reflected back, at this time, the ultrasonic acquisition unit converts the received ultrasonic echo signal into an electrical signal, and performs demodulation, filtering and amplification processing to obtain an original ultrasonic signal. Wherein, the original ultrasonic signal obtained by the controller may be collected by the ultrasonic acquisition unit in real time, or may be stored in the controller, or may be obtained by the controller from other devices, there is no restriction on the source of the original ultrasonic signal herein, which may be specifically set according to actual needs.

The identification of valid signals may also be considered as validity detection, that is, identifying valid signals and invalid signals in the original ultrasonic signal. For the identification of the valid signals, it may be realized by training a valid signal identification model, so that the valid signal identification model learns the features of the valid signals and the invalid signals, so as to be able to identify whether the input signal is a valid signal or not. Alternatively, an envelope signal and a baseline signal may also be obtained by performing envelope and baseline analysis on the original ultrasonic signal, and the valid signals and the invalid signals in the original ultrasonic signal are determined by using the relationship between the envelope signal and the baseline signal.

After distinguishing the valid signals and the invalid signals, the controller processes any invalid signal in the original ultrasonic signal, that is, the controller reduces the amplitude of any invalid signal, and obtains an ultrasonic signal to be processed. It should be noted that the ultrasonic signal to be processed comprises the valid signals in the original ultrasonic signal and the processed invalid signals. The sequence of the valid signals and processed invalid signals has a one-to-one correspondence to the sequence thereof in the original ultrasonic signal.

In some optional implementations of this embodiment, the controller may further perform baseline filtering on the original ultrasonic signal before identifying the valid signals in the original ultrasonic signal. Specifically, white noise is an irregular, zero-mean-value random signal, without obvious periodic peak feature. When the ultrasonic signal does not encounter a motion interface, the frequency shift signal in the echo signal does not have an obvious frequency change, but presents a uniform white noise form. When the ultrasonic signal encounters a motion interface, then it would generate a frequency shift signal with an obvious frequency change. Therefore, the obtained original ultrasonic signal is passed through the baseline filter, if the obtained original ultrasonic signal is white noise, the signal after the baseline filtering is very flat and has no obvious periodic fluctuation peak feature. The baseline filtering processing may adopt a conventional high-low-pass filter, or may be a median filter, a midpoint filter and other nonlinear filters, which is used for removing the influence of Gaussian white noise and interference outside the valid signal frequency band.

S32, Extracting peak features based on the ultrasonic signal to be processed, and determining target gains corresponding to the peak features.

The peak feature extraction may be implemented by using a feature extraction model, or by performing envelope analysis on an ultrasonic signal to be processed, etc., there is no restriction on its implementation herein. Wherein, the peak features comprise, but not limited to, periodicity, peak trend and signal-to-noise ratio, etc. of the ultrasonic signal to be processed.

The peak feature described in this embodiment is not a specific peak feature, but is a general term for all peak features. The controller uses the extracted peak features for calculating the target gains, for example, there is a calculation formula of the target gain built in the controller, after the controller acquires the peak features, the peak features may be substituted into the formula to obtain the corresponding target gains.

Each of the peak features may correspond to each sampling point in an ultrasonic signal to be processed, or may also correspond to all sampling points within one range having a fixed size in the ultrasonic signal to be processed.

When each of the peak features corresponds to each sampling point in the ultrasonic signal to be processed, each of the target gains also corresponds to each sampling point.

When each of the peak features corresponds to all sampling points in one range having a fixed size in the ultrasonic signal to be processed, a mean value or other statistical values of the peak features of the sampling points in the fixed range may be calculated, and then the corresponding target gain may be calculated based on the calculation result. For example, the calculation of the peak features is performed by using a sliding window, and accordingly, the target gains corresponding to the sliding window is obtained.

S33, Performing gain processing on the original ultrasonic signal based on the target gains to determine a target ultrasonic signal;

After determining the target gains, the controller performs gain processing on the corresponding original ultrasonic signal by using the target gains to obtain a final target ultrasonic signal after noise reduction. As mentioned above, each of the target gains may correspond to each sampling point, or may correspond to a plurality of sampling points.

When each of the target gains corresponds to each sampling point, the gain processing is performed for each sampling point; when each of the target gains corresponds to a plurality of sampling points, the gain processing is performed on the plurality of sampling points by using the same target gain.

S34, Analyzing the target ultrasonic signal to determine the fetal heart rate.

For the specific analysis process thereof, please refer to the foregoing, it is not repeatedly described herein.

By performing valid signal identification on the original ultrasonic signal, the valid signal and the invalid signal are distinguished, any invalid signal is firstly processed to obtain an ultrasonic signal to be processed, and then corresponding target gains are determined by using the extracted peak features, so that the obtained target gains correspond to the peak features of the ultrasonic signal to be processed, so that adaptive gain processing can be performed. Since the invalid signal in the signal to be processed is processed firstly to suppress interference, and then the adaptive gain processing is performed, thus, accidental mutation interference can be suppressed, the experience for the detection object is improved, and the noise reduction effect is improved.

In some optional embodiments, the above step S31 may comprise:

S311, Filtering the original ultrasonic signal to obtain an envelope signal and a baseline signal of the original ultrasonic signal.

For the acquisition of the envelope signal and the baseline signal, it may be obtained by a low-pass filter, or by using a smoothing window, etc. Specifically, two low-pass filters with different cut-off frequencies are used for performing low-pass filtering processing on the ultrasonic Doppler signal, one is used for extracting the baseline signal and the other is used for extracting the envelope signal; the ultrasonic Doppler signal may also be smoothed by using two smoothing windows of different sizes, one larger smoothing window is used for acquiring the baseline signal, and the other smaller smoothing window is used for acquiring the envelope signal.

S312, Acquiring a weight corresponding to the baseline signal, and determining a threshold by using the baseline signal and the corresponding weight.

The weight corresponding to the baseline signal is set according to an empirical value, and may also be obtained by analyzing the ultrasonic signal to be processed. After obtaining the weight, the controller calculates a product of the weight and the baseline signal to determine the corresponding threshold.

S313, Based on a size relationship between the envelope signal and the corresponding threshold, determining valid signal and invalid signal in the original ultrasonic signal.

The controller uses the weighted value of the baseline signal as the threshold value, and compares the threshold value with the envelope signal, thereby identifying the valid signal. Wherein, there are many ways to compare the size relationship between the envelope signal and the corresponding threshold, which will be described in the forms of using a difference value and using a ratio respectively.

In some optional implementations of this embodiment, the step S313 described above may comprise:

(1) Calculating a difference value between the envelope signal and the corresponding threshold.

(2) When the difference value is a positive value, it is determined that the signal part corresponding to the positive value in the envelope signal is the valid signal.

The difference signal $L(n)$ is obtained by subtracting the baseline signal $B(n)$ weighted by the weight $W(n)$ from the envelope signal $E(n)$ point by point, that is, $L(n)=E(n)-W(n)*B(n)$, at this time, respective numerical items in the $L(n)$ sequence comprise positive values and non-positive values, and the signals corresponding to the positive values represent the valid signals in the original ultrasonic signal, and conversely, the signals corresponding to the non-positive values represent the invalid signals in the original ultrasonic signal.

In some other optional implementations of this embodiment, the above step S313 may comprise:

(1) Calculating a ratio of the envelope signal to the corresponding threshold.

(2) When the ratio is greater than 1, determining that the signal part corresponding to the ratio that is greater than 1 in the envelope signal is the valid signal.

The signal-to-noise ratio signal R(n) is obtained by using the ratio of the envelope signal E(n) to the baseline signal B(n) weighted by the weight W(n), that is, R(n)=E(n)/(W(n)*B(n)), at this time, respective values in the R(n) sequence comprise values greater than 1 and values not greater than 1, the signals corresponding to the values greater than 1 represent valid signals in the original ultrasonic signal, and conversely, the signals corresponding to the values not greater than 1 represent invalid signals in the original ultrasonic signal.

S314, Processing a signal slope of the invalid signal to obtain the ultrasonic signal to be processed.

After distinguishing the invalid signals in the original ultrasonic signal, the controller may reduce the signal slopes of the invalid signals, thereby reducing the signal fluctuation of the invalid signals and making the invalid signals tend to be flat. Wherein, the processing ways of the signal slopes of the invalid signals is not limited herein, and it is only necessary to ensure that the signal slopes of the invalid signals are reduced.

After processing the signal slopes of the invalid signals by the controller, the invalid signals in the original ultrasonic signal change correspondingly, so that an ultrasonic signal to be processed can be obtained.

As an optional implementation of this embodiment, the above step S314 may comprise:

(1) Setting the signal slope of the invalid signal to zero.

(2) Marking the valid signal by using a first identifier and marking the invalid signal by using a second identifier to obtain the ultrasonic signal to be processed.

After identifying the valid signals, the controller retains the location information of the valid signals in the original ultrasonic signal, and correlates the location information thereof to the coordinates in the original ultrasonic signal, the signal parts corresponding to the valid signals are marked as peak signal sections by using a first identifier, and the other parts are marked as noise signal sections by using a second identifier, and the signal slope values in the noise sections are set to 0 to obtain the ultrasonic signal $S_n(n)$ to be processed.

Because the signal slope of the invalid signal is set to be zero, the invalid signal becomes a constant value, so that the data volume of subsequent gain processing is reduced; and since the valid signal and invalid signal are distinguished and marked by using the corresponding identifiers, the valid signal and the invalid signal can be accurately distinguished from the ultrasonic signal to be processed.

Because the threshold is determined based on the baseline signal and the corresponding weight, that is, the setting of the threshold corresponds to the original ultrasonic signal, and then the envelope signal and the corresponding threshold are used for determining the valid signal and the invalid signal, thus, the reliability of the valid signal recognition can be ensured, thereby ensuring the accuracy of the obtained ultrasonic signal to be processed, and improving the noise reduction effect.

In some other optional embodiments, the above step S32 may comprise:

S321, Determining a periodicity of the ultrasonic signal to be processed based on peak position and peak spacing of the ultrasonic signal to be processed.

During the processing procedure of the step S32, because the envelope signal of the original ultrasonic signal has been obtained, the peak position and peak spacing of the ultrasonic signal to be processed can be determined by using the envelope signal. The periodicity of the ultrasonic signal to be processed is determined by analyzing the peak position and the peak spacing. The periodicity is used for indicating whether the ultrasonic signal to be processed is a periodic signal or an aperiodic signal.

For example, in the ultrasonic scanning process, since the position of the scanning target is unable to be determined at the beginning, the collected original ultrasonic signal is an aperiodic signal, and as the scanning goes on, the scanning position is continuously corrected, so that a more accurate position of the scanning target can be obtained, thereby getting an original ultrasonic signal which is a periodic signal. Wherein, in the scanning process, there may be a temporary aperiodic signal due to the presence of interference. Both the periodic signal and the aperiodic signal can be obtained by analyzing the peak position and the peak spacing of the ultrasonic signal to be processed.

Specifically, the periodicity detection of the peak value is used for checking whether the peak signal segment appears periodically.

If the ultrasonic acquisition unit is not in contact with skin, or although the ultrasonic acquisition unit is in contact with the skin, if the ultrasonic acquisition unit is not moved or the ultrasonic acquisition unit acquires other signals, when the signal acquired by the ultrasonic acquisition unit is processed by the above step S31, a periodic peak value would be unable to be identified in the envelope signal, do it will be identified as a noise signal.

When the ultrasonic acquisition unit encounters a motion interface, it acquires a frequency shift signal, and after the above step S31, the peak position of the signal can be obtained by screening, and then the periodicity of the peaks can be judged according to the peak spacing.

S322, Determining a peak trend feature based on energy change between adjacent windows of the ultrasonic signal to be processed calculated by using sliding windows.

The peak trend feature may determine the steepness and the trend of the peaks according to the slope change of the peak signal. Specifically, the slope change of the ultrasonic signal $S_n(n)$ to be processed is calculated by using the signal energy. That is, firstly, the signal is smoothed by using sliding windows, then the energy mean value of the respective sampling points within one sliding window is calculated, then the energy mean value corresponding to another adjacent window is calculated, and finally the energy change corresponding to the two adjacent sliding windows is calculated. Wherein, the steeper the peak value, the greater the energy value.

S323, Determining a signal-to-noise ratio by using a relationship between the energy corresponding to each sliding window and a baseline signal of the original ultrasonic signal.

The signal-to-noise ratio feature recognition may be done by calculating a ratio of the energy mean value in the above-mentioned sliding window to the baseline signal in step S31 to obtain a signal-to-noise ratio.

S324, Determining the target gains based on the periodicity, the peak trend feature and the signal-to-noise ratio.

As mentioned above, a calculation formula of target gain is set in the controller, after the periodicity, the peak trend feature and the signal-to-noise ratio are obtained by calculating, the target gains may be determined by substituting them into the calculation formula. Specifically, differential continuous control is performed according to a signal trend, amplitude, a signal-to-noise ratio and other peak features, so that the signal amplitude is kept relatively stable, a periodic peak value is highlighted, and base noise and interference are suppressed.

The calculation of target gains may corresponds to each sampling point or may corresponds to a sliding window. Taking the sliding window as an example, the controller calculates the signal slope mean value of all sampling points in the sliding window, as well as the energy value, the amplitude and the signal-to-noise ratio and other peak features corresponding to the sliding window as gain control factors, and uses the above calculation formula to adjust the target gain adaptively in multiple dimensions.

It should be noted that only the target gains corresponding to the valid signal may be calculated, or the target gains corresponding to all the signals to be processed may also be calculated. When only the target gains corresponding to the valid signal are calculated, gain processing is performed on the valid signal by using the target gains, and gain processing or attenuation processing is not performed on the invalid signal; when the target gains corresponding to all signals to be processed are calculated, since the target gains are calculated based on the peak features, and before the peak feature calculation, the signal slope of the invalid signal has been processed, that is, it is equivalent to that the attenuation processing has been performed on the invalid signal. At this time, the gain processing is performed on the ultrasonic signal to be processed by using the corresponding target gains to determine the target ultrasonic signal.

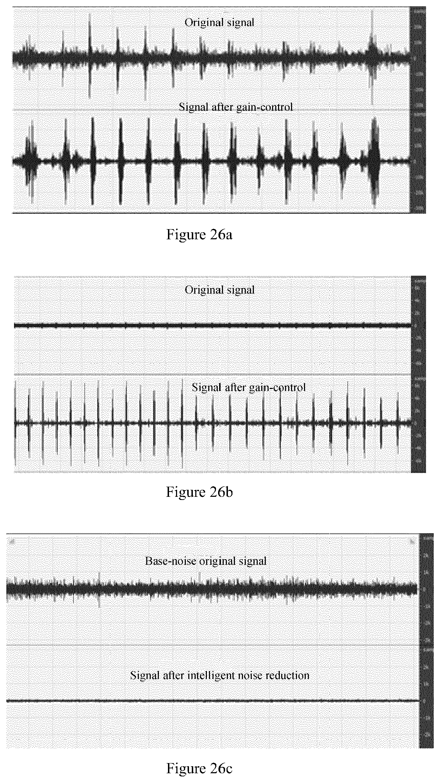

As shown in Figure 26a, the upper graph shows an original ultrasonic signal before automatic gain control, and the lower graph shows a target ultrasonic signal after noise reduction processing by the ultrasonic signal de-noising method provided according to the embodiments of the present disclosure. It can be seen from Figure 26a that the signal amplitude and peak beat feature are significantly enhanced, while the noise signal is significantly suppressed.

As shown in Figure 26b, the upper graph shows an original ultrasonic signal before automatic gain control, and the lower graph shows a target ultrasonic signal after noise reduction processing by the ultrasonic signal de-noising method provided according to the embodiments of the present disclosure. It can be seen from Figure 26b that, even if the amplitude of the original ultrasonic signal fluctuates slightly, the signal amplitude and peak beat feature are significantly enhanced after being processed by the embodiment of the present disclosure, while the noise signal is significantly suppressed.

As shown in Figure 26c, the upper graph shows an original ultrasonic signal before automatic gain control, and the lower graph shows a target ultrasonic signal after noise reduction processing by the ultrasonic signal de-noising method provided according to the embodiments of the present disclosure. It can be seen from Figure 26c that the noise signal is significantly suppressed.

By identifying the periodicity of the ultrasonic signal to be processed, the aperiodic signal and periodic signal can be identified, and in combination with the peak trend feature and the signal-to-noise ratio and other features, the differential continuous control can be performed, so that the signal amplitude is kept relatively stable, the periodic peak value is highlighted, and the base noise and interference are suppressed.

As an optional embodiment of this embodiment, the method further comprises:

(1) Determining prompt information based on the periodicity and the valid signal in the original ultrasonic signal.

(2) Displaying the target ultrasonic signal and the prompt information on an ultrasonic interface.

According to the validity and periodicity of the original ultrasonic signal, different gain control processing procedures are performed to output the target ultrasonic signal, and the corresponding prompt may be given when no continuous stable periodic signal is obtained.

For example, when the valid signal is not identified, it is determined that the prompt information is that no valid signal is identified. If the ultrasonic acquisition unit is not in contact with skin, or although the ultrasonic acquisition unit is in contact with the skin, if the ultrasonic acquisition unit is not moved or the ultrasonic acquisition unit acquires other signals, the peak detection module would be unable to recognize the valid periodic peaks, and would recognize the original ultrasonic signal as a noise signal with a signal slope of 0, and the energy at this time is very small and tends to be zero, then noise suppression is directly performed on the original ultrasonic signal in the gain control processing, that is, the signal will not be amplified or attenuated, and it is prompted that no valid signal is identified.

For another example, when the valid signal is identified and the periodicity has an irregular periodic feature, the prompt information is determined to be that interference is identified. When the ultrasonic acquisition unit acquires a valid signal but the peak values thereof do not have a regular periodic feature, base noise suppression (that is, reducing the signal slope of the invalid signal) is performed on the signal noise section of the ultrasonic signal to be processed, while nonlinear gain amplification is performed on the periodic peak values within the valid signal section, and nonlinear gain control is performed on the aperiodic peak values. At this time, it is prompted that interference is identified.

When the ultrasonic acquisition unit acquires the valid signal and identifies the periodic peak values, gain processing would be performed on the ultrasonic signal to be processed according to the corresponding target gains to obtain the target ultrasonic signal.

Because the corresponding prompt information is displayed on the ultrasonic interface, a detection object can intuitively perceive the currently acquired original ultrasonic signal, so as to guide the subsequent acquisition of the original ultrasonic signal.

In the case that an additional sensor is not required, by performing morphological recognition and nonlinear gain control on the acquired original ultrasonic signal, thereby eliminating the base noise when the ultrasonic acquisition unit is idle and eliminating the self-excited squealing interference of the ultrasonic acquisition unit, suppressing the intermittent aperiodic interference generated by accidental mutation interference such as movement of the ultrasonic acquisition unit, fetal movement, hiccup, etc., and improving the experience of the detection object, at the same time, it can also perform the nonlinear gain control on the original ultrasonic signal to highlight the periodic feature of the signal and improve the signal-to-noise ratio.

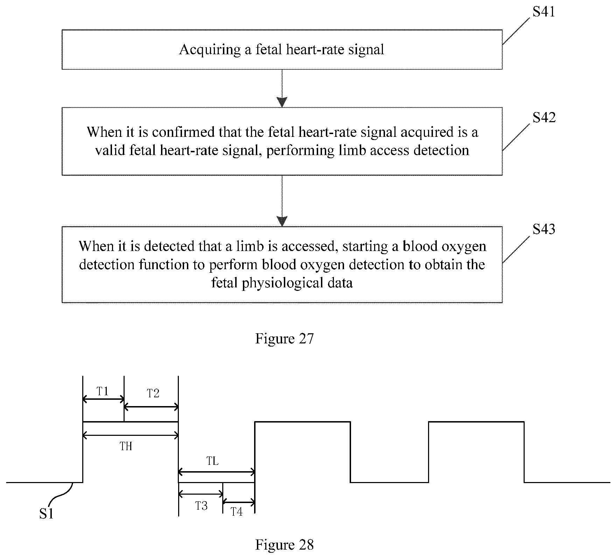

In some optional embodiments, the fetal detection unit in the fetal Doppler comprises an ultrasonic acquisition unit and a blood oxygen acquisition unit. Based on this, the acquired fetal physiological data comprises fetal blood oxygen data, as shown in Figure 27, acquiring fetal physiological data collected by the fetal detection unit of the above step S11 comprises the following steps:

S41, Acquiring a fetal heart-rate signal.

The fetal heart-rate signal refers to a mechanical motion signal formed by systolic and diastolic motions of the heart wall, opening and closing of the heart valves, and movements of the cardiovascular system during heartbeats. The fetal heart-rate signal can be picked up by the ultrasonic acquisition unit, that is, by placing an ultrasonic acquisition unit equipped with an ultrasonic transmitter and an ultrasonic receiver on the surface of the maternal abdomen, and aligning it with the fetal heart, sending the ultrasonic wave, and receiving the reflected ultrasonic echo. The frequency of the ultrasonic echo is modulated by the moving heart, that is, the angular frequency of the Doppler frequency shift is proportional to the speed of the systolic and diastolic movements of the heart or the blood vessel wall, a fetal heart-rate signal can be obtained by demodulation.

S42, When it is confirmed that the fetal heart-rate signal acquired is a valid fetal heart-rate signal, performing limb access detection.

The controller performs limb access detection after determining that the fetal heart-rate signal acquired is a valid fetal heart-rate signal by performing valid signal analysis on the fetal heart-rate signal. For the analysis of the valid fetal heart-rate signal, whether the fetal heart-rate signal is a valid fetal heart-rate signal or not may be determined by analyzing the energy of the fetal heart-rate signal and comparing the energy value with an energy threshold; or the fetal heart-rate signal and the baseline signal may also be compared and analyzed to determine whether the fetal heart-rate signal is an valid fetal heart-rate signal or not.

In some optional embodiments, confirming that the fetal heart-rate signal acquired is a valid fetal heart-rate signal of the above step S42 may comprise:

S421, Judging whether the fetal heart-rate signal is greater than a preset signal threshold;

S422, If the fetal heart-rate signal is greater than the preset signal threshold, judging whether the fetal heart-rate signal satisfies a preset condition;

S423, When the fetal heart rate signal satisfies the preset condition, determining that the fetal heart-rate signal acquired is a valid fetal heart-rate signal.

In a specific embodiment, it may be judged whether the fetal heart-rate signal satisfies a preset condition, when the fetal heart-rate signal satisfies the preset condition, it is determined that the fetal heart-rate signal acquired is a valid fetal heart-rate signal.

Further optionally, the above step S422 may comprise:

(1) Performing a first filtering processing on an absolute value signal of the fetal heart-rate signal to obtain a first signal, and extracting an envelope signal of the first signal.

In this embodiment, by connecting the peak points of the fetal heart-rate signal over a period of time, an upper (positive) line and a lower (negative) line can be obtained, these two lines are called envelope lines. The envelope lines form a curve reflecting the amplitude variation of the fetal heart-rate signal.

(2) Performing a second filtering processing on the absolute value signal of the fetal heart-rate signal to obtain a second signal, and extracting a baseline signal of the second signal.

Optionally, the first and second filtering processing are performed on the absolute value signal of the fetal heart-rate signal by using a low-pass digital filter, and the cut-off frequency of the first filtering processing is set to be greater than the cut-off frequency of the second filtering processing.

The fetal heart-rate baseline signal refers to a fetal heart-rate average value over 10 minutes without fetal movement and uterine contraction. In this embodiment, the normal fetal heart-rate baseline is 110 ~ 160 bpm, when the fetal heart-rate baseline is greater than 160 bpm, it indicates fetal tachycardia. When the fetal heart-rate baseline is less than 110bpm, it indicates fetal bradycardia. It should be noted that the fetal heart-rate baseline may be obtained by ways of mean value or low-pass filtering.

(3) Within a preset time range, if a ratio of the envelope signal to the baseline signal is greater than a preset signal quality threshold, determining that the fetal heart-rate signal satisfies the preset condition.

In this embodiment, since the fetal heart-rate signal received by the ultrasonic acquisition unit for detecting the fetal heart-rate signal may contain a lot of noise signals, the fetal heart-rate signal may be filtered (low-pass), thereby obtaining a noise-free fetal heart-rate signal.

Since the fetal heart-rate can be stably calculated only when the valid fetal heart-rate signal is acquired by the ultrasonic acquisition unit, in this embodiment, the validity of the current fetal heart-rate signal may also be extrapolated from the stability of the calculated fetal heart-rate values.

Further, if the light-emitting diode is turned on immediately after the reflective blood oxygen device is started, when the device is idle or before the limb is normally accessed, the light-emitting diode would work all the time, so the service life of the light-emitting diode would be greatly shortened, the power consumption of the device would be increased, and meanwhile, the light emitted by the light-emitting diode would also bring discomfort to human eyes. If the light-emitting diode is not started immediately, the light-emitting diode needs to be turned on by a second manual operation after the device is started, which increases the complexity of the operation.

In view of this, in some optional embodiments, performing limb access detection of the above step S42 may comprise:

S421, Controlling an infrared light tube and/or a red light tube to be turned on or turned off according to a first preset timing sequence. Wherein, the infrared light tube and the red light tube are not turned on at the same time.

Specifically, a first pulse control signal is output to the infrared light tube and/or the red light tube.

Wherein, referring to Figure 28, Figure 28 is a pulse signal schematic diagram of the first pulse control signal in this embodiment. As shown in Figure 28, the red light tube (not shown in the drawings) is configured to be turned off at the beginning of a rising edge of the first pulse control signal S1, and be turned on after delaying for a first preset time period T1, and continue being on for a second preset time period T2, a sum of the first preset time period T1 and the second preset time period T2 is a high-level duration TH of the first pulse control signal S1.

The infrared light tube (not shown in the drawings) is configured to be turned off at the beginning of a falling edge of the first pulse control signal S1, and be turned on after delaying for a third preset time period T3, and continue being on for a fourth preset time period T4, a sum of the third preset time period T3 and the fourth preset time period T4 is a low-level duration TL of the first pulse control signal S1.

S422, Collecting infrared light and/or red light.

S423, Judging whether there is a limb accessed according to the signal strength of the infrared light and/or the signal strength of the red light.

Specifically, the above step S423 may comprise:

S4231, Determining a first amplitude of the infrared light collected during a turned-on time period of the infrared light tube, and determining a second amplitude of the infrared light collected during a turned-off time period of the infrared light tube. And/or, determining a third amplitude of the red light collected during a turned-on time period of the red light tube, and determining a fourth amplitude of the red light collected during a turned-off time period of the red light tube.

S4232, When the amplitude difference between the first amplitude and the second amplitude is greater than a preset first amplitude threshold and/or the amplitude difference between the third amplitude and the fourth amplitude is greater than a preset second amplitude threshold, determining that there is a limb accessed.

In this specific embodiment, if there is no limb accessed, since there is no limb reflection, the receiving end is basically unable to receive the red light or infrared light emitted by the red light tube or infrared light tube, and can only receive the red light or infrared light in the environment. Therefore, in this case, the first amplitude acquired in the turned-on time period of the infrared light tube is basically equal to the second amplitude acquired in the turned-off time period of the infrared light tube, thus, when the amplitude difference between the first amplitude and the second amplitude is less than a preset first amplitude threshold, it is determined that there is no limb accessed. Similarly, the third amplitude obtained in the turned-on time period of the red light is basically equal to the fourth amplitude obtained in the turned-off time period of the red light, thus, when the amplitude difference between the third amplitude and the fourth amplitude is less than a preset second amplitude threshold, it is determined that there is no limb accessed.

It can be understood that the first amplitude threshold in this embodiment is related to the infrared light absorption capacity of red blood cells in the blood of the limb to be detected. More specifically, the stronger the infrared light absorption capacity of red blood cells is, the weaker the infrared light signal received by the receiving end is, and thus it is closer to the infrared light signal in the ambient light, which would require the first amplitude threshold to be set smaller; and for the opposite situation, the first amplitude threshold would be required to be set larger. Similarly, the second amplitude threshold may also be determined in the above manner, which is not redundantly described herein.

In another specific embodiment, the above step S423 may comprise:

S4233, Determining a first energy of infrared light emitted by the infrared light tube and a second energy of the collected infrared light during a turned-on time period of the infrared light tube. And/or determining a third energy of red light emitted by the red light tube and a fourth energy of the collected red light during a turned-on time period of the red light tube.
S4234, When a ratio of the second energy to the first energy is greater than a preset first proportion threshold and/or a ratio of the third energy to the fourth energy is greater than a preset second proportion threshold, determining that there is a limb accessed.

In this embodiment, in the case that there is no limb accessed, since there is no limb reflection, the receiving end is basically unable to receive the red light or infrared light emitted by the red light tube or infrared light tube, and can only receive the red light or infrared light in the environment. Therefore, in the case that there is no limb accessed, the second energy of the collected infrared light is the energy of the infrared light in the environment. It can be understood that, under the condition that the first energy of the infrared light emitted by the infrared light tube during the turned-on time period of the infrared light tube is not changed, the ratio of the second energy to the first energy when there is no limb accessed is smaller than the ratio thereof when there is a limb accessed, that is, if the ratio of the second energy to the first energy is greater than the preset first proportion threshold, it is determined that there is a limb accessed.

Similarly, during the turned-on time period of the red light tube, whether there is a limb accessed may be judged by calculating the ratio of the fourth energy of the collected red light to the third energy of the red light emitted by the red light tube.

In this embodiment, since the energy of the infrared light or red light in the ambient light is different in strong light environment and in weak light environment, as described above, in the case that there is no limb accessed, the energy of the collected infrared light or red light is the energy of the infrared light or red light in the environment. Therefore, in the turned-on time period of the infrared light tube, under the condition that the first energy of the infrared light emitted by the infrared light tube does not change, the infrared light energy in the ambient light that determines the amount of the second energy is related to the intensity of the ambient light.

Therefore, in this embodiment, the first proportional threshold is determined by the intensity of the infrared light collected during the turned-off time period of the infrared light tube. The second proportional threshold is determined by the intensity of the red light collected during the turn-off time period of the red light tube. More specifically, if the infrared light intensity/red light intensity is stronger, the first proportional threshold/second proportional threshold can be set larger; and for the opposite situation, the first proportion threshold/the second proportion threshold can be set smaller.

Optionally, in this embodiment, if only the valid fetal heart-rate signal is detected and the limb access is not detected, limb access detection is repeated, that is, controlling the infrared light tube and/or the red light tube to be turned on or turned off according to a first preset timing sequence, and continue to wait for a limb to be accessed according to the variation of the received signal intensity of the red light and/or the received signal intensity of the infrared light.

S43, When it is detected that a limb is accessed, starting a blood oxygen detection function to perform blood oxygen detection to obtain the fetal physiological data.

Specifically, the above step S43 may comprise: S431, Controlling the infrared light tube and/or the red light tube to be turned on or turned off according to a second preset timing sequence. Wherein, the infrared light tube and the red light tube are not turned on at the same time.

Generally, a dual-wavelength transmission photoelectric detection method is used for measuring the blood oxygen saturation, wherein a lightless condition, 660 nm red light and 940 nm infrared light may be alternately generated, and signals of different lighting time periods are collected, the influence of ambient light may be removed by subtracting the electric signal of the lightless time period from the signals of different lighting time periods, and the ratio of the alternating component of red light to the alternating component of infrared light caused by light absorption by arterial blood after removing the influence of ambient light can be obtained.

Specifically, in this embodiment, the red light tube is configured to be turned off at the beginning of a rising edge of the second pulse control signal, and be turned on after delaying for a fifth preset time period, and continuing being on for a sixth preset time period, a sum of the fifth preset time period and the sixth preset time period is a high-level duration of the second pulse control signal.

The infrared light tube is configured to be turned off at the beginning of a falling edge of the second pulse control signal, and be turned on after delaying for a seventh preset time period, and continuing being on for an eighth preset time period, a sum of the seventh preset time period and the eighth preset time period is a low-level duration of the second pulse control signal.

In this embodiment, the switching frequency corresponding to the second preset timing sequence may be set to be greater than the switching frequency corresponding to the first preset timing sequence, so that the blood oxygen detection time can be correspondingly reduced, the detection accuracy is further enhanced, and the detection speed is increased. In addition, since the first preset timing sequence is used for detecting whether there is a limb accessed, in order to reduce power consumption, it is reasonable to set the switching frequency of the first preset timing sequence to be relatively low.

S432, Performing blood oxygen detection based on the received infrared light and/or red light.

In this embodiment, after the signal containing the blood oxygen saturation is collected, since the acquired signal is weak and contains some noise, the acquired signal needs to be amplified and filtered, and the blood oxygen saturation of the limb is obtained according to the processed signal.

Optionally, in this embodiment, when the detected fetal heart-rate signal is an invalid fetal heart-rate signal, or when the detected fetal heart-rate signal is an valid fetal heart-rate signal but no limb access is detected, the step S43 would not be performed, that is, the infrared light tube and/or the red light tube would not be controlled to be turned on or turned off according to the second preset timing sequence, and blood oxygen detection would not be performed.

In this method, if the detected fetal heart-rate signal is valid and limb access is detected, blood oxygen detection is performed on the limb, otherwise blood oxygen detection is not performed. To detect blood oxygen, it requires turning on the red light tube or the infrared light tube firstly to transmit red light or infrared light to the limb. Therefore, in fact, the detection method provided in this embodiment can control the starting time point of the red light tube or infrared light tube starting to emit light under the condition that the fetal heart-rate signal is detected to be an valid fetal heart-rate signal and the limb access is detected, thereby reducing power consumption, and also reducing light discomfort caused by blood oxygen detection being on for a long time.

## Claims

1. A fetal Doppler, comprising:

   a housing (1);
   a fetal detection unit (20) and a maternal parameter detection unit (30), wherein the fetal detection unit (20) and the maternal parameter detection unit (30) are respectively used for collecting fetal physiological data and maternal physiological data;
   a controller (7) is arranged in an internal cavity of the housing (1);
   the controller (7) is used for analyzing the collected fetal physiological data and maternal physiological data, so as to obtain a fetal heart rate and a maternal parameter;
   **characterized in that**,
   the fetal detection unit (20) comprises an ultrasonic acquisition unit (2), and the ultrasonic acquisition unit (2) comprises a wafer component (21) having a plurality of wafers and an angle adjustment component (22) for adjusting a signal transmission direction between each of the wafers and a target body;

   the controller (7) is connected to the angle adjustment component (22), and the controller (7) is used for controlling the action of the angle adjustment component (22) on the basis of the signal strength corresponding to each of the wafers, so as to realize target following.

2. The fetal Doppler as claimed in claim 1, **characterized in that**,

   the maternal parameter detection unit (30) is a blood oxygen acquisition unit, the maternal physiological data is a blood oxygen signal, and when the controller (7) is used for analyzing the collected maternal physiological data to obtain the maternal parameter, the controller (7) is further used for: determining an envelope of the blood oxygen signal, and obtaining the maternal parameter based on the envelope;
   or,
   the maternal parameter detection unit (30) is a piezoelectric ceramic wafer, the maternal physiological data is an ultrasonic echo signal, and when the controller (7) is used for analyzing the collected maternal physiological data to obtain the maternal parameter, the controller (7) is further used for: acquiring a maximum frequency envelope signal in the ultrasonic echo signal, and obtaining the maternal parameter based on the maximum frequency envelope signal;
   or,

the maternal parameter detection unit (30) is an ECG electrode, the maternal physiological data is an ECG signal, and when the controller (7) is used for analyzing the collected maternal physiological data to obtain the maternal parameter, the controller (7) is further used for: analyzing the ECG signal to obtain the maternal parameter.

3. The fetal Doppler as claimed in claim 1, **characterized in that**, the maternal parameter detection unit (30) comprises a blood oxygen acquisition unit (3), the blood oxygen acquisition unit (3) is equipped with a light guide structure installed on the housing (1); the housing (1) is provided with a light transmitting window (111); the blood oxygen acquisition unit comprises a light emitting element (31) and a light receiving element (32) arranged on the controller (7), and the light guide structure comprises a first light guide member installed on the housing (1) and located on a light transmission path between the light emitting element (31) and the light transmitting window (111), the first light guide member is configured to transmit light emitted by the light emitting element (31) to the outside of the light transmitting window (111) after being propagated by the first light guide member; the light guide structure further comprises a first barrier arranged in the housing (1) and located between the light transmitting window (111) and the controller (7), wherein a first optical path channel is provided inside the first barrier, the first light guide member is a first light guide column (33) arranged in the first optical path channel.

4. The fetal Doppler as claimed in claim 3, **characterized in that**, the light guide structure further comprises a second light guide member arranged in the housing (1) and located on a light transmission path between the light receiving element (32) and the light transmitting window (111), and the second light guide member is configured to transmit light incident from the light transmitting window (111) to the light receiving element (32) after being propagated by the second light guide; the light guide structure further comprises a second barrier arranged in the housing (1) and located between the light transmitting window (111) and the controller (7), a second optical path channel is provided inside the second barrier, and the second light guide member is a second light guide column (34) arranged in the second optical path channel.

5. The fetal Doppler as claimed in claim 4, **characterized in that**, the first barrier and the second barrier are integrally formed by a blocking base (35), the first optical path channel is a first through hole provided in the blocking base (35), the second optical path channel is a second through hole provided in the blocking base (35).

6. The fetal Doppler as claimed in claim 3, **characterized in that**, the first light guide member is a condensing lens capable of converging light into a light beam, the condensing lens comprises at least one convex lens, and one surface of the convex lens at a side facing the light emitting element (31) is a convex surface.

7. The fetal Doppler as claimed in any one of claims 3-6, **characterized in that**, a fetal heart-rate acquisition surface of the ultrasonic acquisition unit (2) and a blood oxygen acquisition surface of the blood oxygen acquisition unit are located on two different surfaces of the housing (1); the blood oxygen acquisition unit (3) comprises a blood oxygen measurement module for acquiring a blood oxygen signal and a trigger sensing module arranged on the blood oxygen acquisition surface and electrically connected to the controller; when the trigger sensing module senses that there is an obstructing object near the blood oxygen acquisition surface, a control signal for controlling the blood oxygen measurement module to start is sent to the controller.

8. The fetal Doppler as claimed in claim 1, **characterized in that**, ,
the plurality of wafers are divided into central wafers (211) and peripheral wafers (212), the peripheral wafers (212) are arranged around a periphery of the central wafers (211), the controller (7) is used for controlling the action of the angle adjustment component (22) on the basis of the signal strength corresponding to the central wafers (211) and the signal strength corresponding to the peripheral wafers (212).

9. A detection method of a fetal Doppler, wherein the fetal Doppler comprises a fetal detection unit (20) and a maternal parameter detection unit (30), the detection method comprises:

acquiring fetal physiological data collected by the fetal detection unit (20) and maternal physiological data collected by the maternal parameter detection unit (30);
analyzing the collected fetal physiological data and maternal physiological data to obtain a fetal heart rate and a maternal parameter;
**characterized in that**,
the fetal detection unit (20) comprises an ultrasonic acquisition unit (2), the ultrasonic acquisition unit (2) comprises a plurality of wafers and an angle adjustment component (22); the step of acquiring the fetal physiological data collected by the fetal detection unit (20) comprises:

acquiring a signal strength corresponding to each of the wafers;
based on the signal strength corresponding to each of the wafers, controlling an action of the angle adjustment component (22) to realize target tracking, wherein the angle adjustment component (22) is used for adjusting a signal transmission direction between each of the wafers and a target body;
acquiring the fetal physiological data collected by each of the wafers based on the adjusted signal transmission direction.

**10.** The detection method as claimed in claim 9, **characterized in that**, the maternal parameter comprises a maternal heart rate, the detection method further comprises:

detecting whether there is an overlapping interval between the fetal heart rate and the maternal heart rate;
if the overlapping interval is detected, sending a prompt and/or marking the overlapping interval on a displayed heart rate curve, wherein, the heart rate curve comprises at least one of the following: a fetal heart rate curve generated from the fetal heart rate and a maternal heart rate curve generated from the maternal heart rate.

**11.** The detection method as claimed in claim 10, **characterized in that**, the step of detecting whether there is an overlapping interval between the fetal heart rate and the maternal heart rate comprises:

acquiring a first number of heart rate differences respectively corresponding to different moments within a first time period, wherein, the heart rate difference corresponding to each moment is a difference feature value between the fetal heart rate and the maternal heart rate at the moment;
counting a second number of the heart rate differences satisfying a first condition within the first time period, the first condition is that the heart rate difference is less than a preset threshold;
if the second number satisfies a second condition and the heart rate differences within a second time period in the first time period all satisfies the first condition, determining that the first time period is an overlapping interval between the fetal heart rate and the maternal heart rate, the second condition is that the proportion of the second number in the first number is greater than a preset proportion.

**12.** The detection method as claimed in claim 9, **characterized in that**, the plurality of wafers are divided into central wafers and peripheral wafers, the peripheral wafers are arranged around a periphery of the central wafers, the step of, based on the signal strength corresponding to each of the wafers, controlling the action of the angle adjustment component (22) to realize target following comprises:

comparing the signal strength of the plurality of wafers, and judging whether a wafer having the strongest signal strength is a central wafer;
when the wafer having the strongest signal strength is a peripheral wafer, calculating a signal strength difference value between the signal strength of the central wafers and the strongest signal strength;
based on the signal strength difference value and the position of the wafer having the strongest signal strength, determining an adjustment angle and direction of the angle adjustment component (22).

**13.** The detection method as claimed in any one of claims 9-12, **characterized in that**, when the fetal physiological data is an original ultrasonic signal, the step of analyzing the collected fetal physiological data to obtain a fetal heart rate comprises:

performing valid signal identification on the original ultrasonic signal, and processing any invalid signal in the original ultrasonic signal to obtain an ultrasonic signal to be processed;
extracting peak features based on the ultrasonic signal to be processed, and determining target gains corresponding to the peak features;
performing gain processing on the original ultrasonic signal based on the target gains to determine a target ultrasonic signal;
analyzing the target ultrasound signal to determine the fetal heart rate.

**14.** The detection method as claimed in claim 9, **characterized in that**, when the fetal detection unit (20) comprises an ultrasound acquisition unit and a blood oxygen acquisition unit, the step of acquiring fetal physiological data collected by the fetal detection unit (20) comprises:

acquiring a fetal heart-rate signal;
when it is confirmed that the fetal heart-rate signal acquired is a valid fetal heart-rate signal, performing limb access detection;
when it is detected that a limb is accessed, starting a blood oxygen detection function to perform blood oxygen detection to obtain the fetal physiological data.

## Patentansprüche

1. Fetaldoppler, umfassend:

ein Gehäuse (1);
eine fetale Erfassungseinheit (20) und eine Erfassungseinheit für maternale Parameter (30), wobei die fetale Erfassungseinheit (20) und die Erfassungseinheit für maternale Parameter (30) jeweils verwendet werden, um physiologische Daten des Fötus und physiologische Daten der Mutter zu sammeln;
eine Steuerung (7) ist in einem Innenhohlraum des Gehäuses (1) angeordnet;
die Steuerung (7) wird verwendet zum Analysieren der gesammelten physiologischen Daten des Fötus und der physiologischen Daten der Mutter, um eine fetale Herzfrequenz und einen maternalen Parameter zu erhalten;
**dadurch gekennzeichnet, dass**
die fetale Erfassungseinheit (20) eine Ultraschallerfassungseinheit (2) umfasst und die Ultraschallerfassungseinheit (2) eine Wafer-Komponente (21) mit einer Vielzahl von Wafern und eine Winkeleinstellkomponente (22) zum Anpassen einer Signalübertragungsrichtung zwischen jedem der Wafer und einem Zielkörper umfasst;
die Steuerung (7) ist mit der Winkeleinstellkomponente (22) verbunden und die Steuerung (7) wird verwendet zum Steuern der Wirkung der Winkeleinstellkomponente (22) basierend auf der Signalstärke entsprechend jedem der Wafer, um Zielverfolgung zu realisieren.

2. Fetaldoppler nach Anspruch 1, **dadurch gekennzeichnet, dass**

die Erfassungseinheit für maternale Parameter (30) eine Blutsauerstoff-Erfassungseinheit ist, die physiologischen Daten der Mutter ein Blutsauerstoffsignal sind und wenn die Steuerung (7) zum Analysieren der gesammelten physiologischen Daten der Mutter verwendet wird, um den maternalen Parameter zu erhalten, die Steuerung (7) ferner für Folgendes verwendet wird: Bestimmen einer Hüllkurve des Blutsauerstoffsignals und Erhalten der maternalen Parameter basierend auf der Hüllkurve;
oder
die Erfassungseinheit für maternale Parameter (30) ein piezoelektrischer Keramikwafer ist, die physiologischen Daten der Mutter ein Ultraschallechosignal sind und wenn die Steuerung (7) zum Analysieren der gesammelten physiologischen Daten der Mutter verwendet wird, um den maternalen Parameter zu erhalten, die Steuerung (7) ferner für Folgendes verwendet wird: Erfassen eines Hüllkurvensignals mit maximaler Frequenz in dem Ultraschallechosignal und Erhalten des maternalen Parameters basierend auf dem Hüllkurvensignal mit maximaler Frequenz;
oder
die Erfassungseinheit für maternale Parameter (30) eine EKG-Elektrode ist, die physiologischen Daten der Mutter ein EKG-Signal sind und wenn die Steuerung (7) zum Analysieren der gesammelten physiologischen Daten der Mutter verwendet wird, um die maternalen Parameter zu erhalten, die Steuerung (7) ferner für Folgendes verwendet wird: Analysieren des EKG-Signals, um den maternalen Parameter zu erhalten.

3. Fetaldoppler nach Anspruch 1, **dadurch gekennzeichnet, dass** die Erfassungseinheit für maternale Parameter (30) eine Blutsauerstoff-Erfassungseinheit (3) umfasst, die Blutsauerstoff-Erfassungseinheit (3) mit einer Lichtleiterstruktur ausgestattet ist, die an dem Gehäuse (1) installiert ist; das Gehäuse (1) mit einem lichtdurchlässigen Fenster (111) bereitgestellt ist; die Blutsauerstoff-Erfassungseinheit ein lichtemittierendes Element (31) und ein lichtempfangendes Element (32) umfasst, das an der Steuerung (7) angeordnet ist, und die Lichtleiterstruktur ein erstes Lichtleiterelement umfasst, das an dem Gehäuse (1) installiert ist und sich an einem Lichtübertragungsweg zwischen dem lichtemittierenden Element (31) und dem lichtdurchlässigen Fenster (111) befindet; das erste Lichtleiterelement konfiguriert ist, um Licht, das durch das lichtemittierende Element (31) emittiert wird, außerhalb des lichtdurchlässigen Fensters (111) zu übertragen, nachdem es durch das erste Lichtleiterelement propagiert wird; die Lichtleiterstruktur ferner eine erste Grenze umfasst, die in dem Gehäuse (1) angeordnet ist und sich zwischen dem lichtdurchlässigen Fenster (111) und der Steuerung (7) befindet, wobei ein erster Strahlengangkanal innerhalb der ersten Grenze bereitgestellt ist, das erste Lichtleiterelement eine erste Lichtleitersäule (33) ist, die in dem ersten Strahlengangkanal angeordnet ist.

4. Fetaldoppler nach Anspruch 3, **dadurch gekennzeichnet, dass** die Lichtleiterstruktur ferner ein zweites Lichtleiterelement umfasst, das in dem Gehäuse (1) angeordnet ist und sich auf einem Lichtübertragungsweg zwischen dem lichtempfangenden Element (32) und dem lichtdurchlässigen Fenster (111) befindet, und das zweite Lichtleiterelement konfiguriert ist, um Licht, das von dem lichtdurchlässigen Fenster (111) einfällt, an das lichtempfangende Element (32) zu übertragen, nachdem es durch den zweiten Lichtleiter propagiert wird; die Lichtleiterstruktur ferner eine zweite Grenze umfasst, die in dem Gehäuse (1) angeordnet ist und sich zwischen dem lichtdurchlässigen Fenster (111) und der Steuerung (7) befindet, ein zweiter Strahlengangkanal innerhalb der zweiten Grenze bereitgestellt ist und das zweite Lichtleiterelement eine zweite Lichtleitersäule (34) ist, die in dem zweiten Strahlengangkanal angeordnet ist.

**5.** Fetaldoppler nach Anspruch 4, **dadurch gekennzeichnet, dass** die erste Grenze und die zweite Grenze durch eine blockierende Basis (35) einstückig gebildet sind, der erste Strahlengangkanal ein erstes Durchgangsloch ist, das in der blockierenden Basis (35) bereitgestellt ist, der zweite Strahlengangkanal ein zweites Durchgangsloch ist, das in der blockierenden Basis (35) bereitgestellt ist.

**6.** Fetaldoppler nach Anspruch 3, **dadurch gekennzeichnet, dass** das erste Lichtleiterelement eine Kondensorlinse ist, die in der Lage ist, Licht in einen Lichtstrahl zu konvergieren, die Kondensorlinse mindestens eine Konvexlinse umfasst und eine Oberfläche der Konvexlinse auf einer Seite, die dem lichtemittierenden Element (31) zugewandt ist, eine konvexe Oberfläche ist.

**7.** Fetaldoppler nach einem der Ansprüche 3-6, **dadurch gekennzeichnet, dass** sich eine Erfassungsfläche für fetale Herzfrequenz der Ultraschallerfassungseinheit (2) und eine Blutsauerstoff-Erfassungsfläche der Blutsauerstoff-Erfassungseinheit auf zwei unterschiedlichen Oberflächen des Gehäuses (1) befinden; die Blutsauerstoff-Erfassungseinheit (3) ein Blutsauerstoff-Messmodul zum Erfassen eines Blutsauerstoffsignals und ein Trigger-Erfassungsmodul umfasst, das an der Blutsauerstoff-Erfassungsfläche angeordnet ist und mit der Steuerung elektrisch verbunden ist; wenn das Trigger-Erfassungsmodul erfasst, dass es nahe der Blutsauerstoff-Erfassungsfläche ein behinderndes Objekt gibt, ein Steuersignal zum Steuern des Blutsauerstoff-Messmoduls, sodass es startet, an die Steuerung gesendet wird.

**8.** Fetaldoppler nach Anspruch 1, **dadurch gekennzeichnet, dass**

die Vielzahl von Wafern in zentrale Wafer (211) und periphere Wafer (212) aufgeteilt wird, die peripheren Wafer (212) um eine Peripherie der zentralen Wafer (211) angeordnet sind, die Steuerung (7) verwendet wird zum Steuern der Wirkung der Winkeleinstellkomponente (22) basierend auf der Signalstärke, die den zentralen Wafern (211) entspricht, und der Signalstärke, die den peripheren Wafern (212) entspricht.

**9.** Erfassungsverfahren eines Fetaldopplers, wobei der Fetaldoppler eine fetale Erfassungseinheit (20) und eine Erfassungseinheit für maternale Parameter (30) umfasst, wobei das Erfassungsverfahren Folgendes umfasst:

Erfassen von physiologischen Daten des Fötus, die durch die fetale Erfassungseinheit (20) gesammelt werden, und physiologischen Daten der Mutter, die durch die Erfassungseinheit für maternale Parameter (30) gesammelt werden;
Analysieren der gesammelten physiologischen Daten des Fötus und der physiologischen Daten der Mutter, um eine fetale Herzfrequenz und einen maternalen Parameter zu erhalten;
**dadurch gekennzeichnet, dass**
die fetale Erfassungseinheit (20) eine Ultraschallerfassungseinheit (2) umfasst, die Ultraschallerfassungseinheit (2) eine Vielzahl von Wafern und eine Winkeleinstellkomponente (22) umfasst; der Schritt des Erfassens der physiologischen Daten des Fötus, die durch die fetale Erfassungseinheit (20) gesammelt werden, Folgendes umfasst:

Erfassen einer Signalstärke, die jedem der Wafer entspricht;
basierend auf der Signalstärke, die jedem der Wafer entspricht, Steuern einer Wirkung der Winkeleinstellkomponente (22), um Zielverfolgung zu realisieren, wobei die Winkeleinstellkomponente (22) zum Anpassen einer Signalübertragungsrichtung zwischen jedem der Wafer und einem Zielkörper verwendet wird;
Erfassen der physiologischen Daten des Fötus, die durch jeden der Wafer gesammelt werden, basierend auf der angepassten Signalübertragungsrichtung.

**10.** Erfassungsverfahren nach Anspruch 9, **dadurch gekennzeichnet, dass** der maternale Parameter eine maternale Herzfrequenz umfasst, wobei das Erfassungsverfahren ferner Folgendes umfasst:

Erfassen, ob es überlappendes Intervall zwischen der fetalen Herzfrequenz und der maternalen Herzfrequenz gibt;
wenn das überlappende Intervall erfasst wird, Senden einer Aufforderung und/oder Kennzeichnen des überlappenden Intervalls auf einer angezeigten Herzfrequenzkurve, wobei die Herzfrequenzkurve mindestens eines aus Folgendem umfasst: eine fetale Herzfrequenzkurve, die aus der fetalen Herzfrequenz generiert wird, und eine maternale Herzfrequenzkurve, die aus der maternalen Herzfrequenz generiert wird.

**11.** Erfassungsverfahren nach Anspruch 10, **dadurch**

gekennzeichnet, dass der Schritt des Erfassens, ob es ein überlappendes Intervall zwischen der fetalen Herzfrequenz und der maternalen Herzfrequenz gibt, Folgendes umfasst:

Erfassen einer ersten Anzahl von Herzfrequenzdifferenzen, die jeweils unterschiedlichen Momenten in einem ersten Zeitraum entsprechen, wobei die Herzfrequenzdifferenz, die jedem Moment entspricht, ein Differenzmerkmalswert zwischen der fetalen Herzfrequenz und der maternalen Herzfrequenz zu dem Moment ist;
Zählen einer zweiten Anzahl der Herzfrequenzdifferenzen, die eine erste Bedingung erfüllen, in dem ersten Zeitraum, wobei die erste Bedingung ist, dass die Herzfrequenzdifferenz niedriger als ein voreingestellter Schwellenwert ist;
wenn die zweite Anzahl eine zweite Bedingung erfüllt und die Herzfrequenzdifferenzen innerhalb eines zweiten Zeitraums in dem ersten Zeitraum alle die erste Bedingung erfüllen, Bestimmen, dass der erste Zeitraum ein überlappendes Intervall zwischen der fetalen Herzfrequenz und der maternalen Herzfrequenz ist, wobei die zweite Bedingung ist, dass der Anteil der zweiten Anzahl in der ersten Anzahl größer als ein voreingestellter Anteil ist.

**12.** Erfassungsverfahren nach Anspruch 9, **dadurch gekennzeichnet, dass** die Vielzahl von Wafern in zentrale Wafer und periphere Wafer aufgeteilt ist, die peripheren Wafer um eine Peripherie der zentralen Wafer angeordnet sind, der Schritt des, basierend auf der Signalstärke, die jedem der Wafer entspricht, Steuerns der Wirkung der Winkeleinstellkomponente (22), um Zielverfolgung zu realisieren, Folgendes umfasst:

Vergleichen der Signalstärke der Vielzahl von Wafern und Beurteilen, ob ein Wafer mit der stärksten Signalstärke ein zentraler Wafer ist;
wenn der Wafer mit der stärksten Signalstärke ein peripherer Wafer ist, Berechnen eines Signalstärke-Differenzwerts zwischen der Signalstärke der zentralen Wafer und der stärksten Signalstärke;
basierend auf dem Signalstärke-Differenzwert und der Position des Wafers mit der stärksten Signalstärke Bestimmen eines Einstellwinkels und einer Richtung der Winkeleinstellkomponente (22).

**13.** Erfassungsverfahren nach einem der Ansprüche 9-12, **dadurch gekennzeichnet, dass**, wenn die physiologischen Daten des Fötus ein ursprüngliches Ultraschallsignal sind, der Schritt des Analysierens der gesammelten physiologischen Daten des Fötus, um eine fetale Herzfrequenz zu erhalten, Folgendes umfasst:

Durchführen der Identifikation von gültigem Signal an dem ursprünglichen Ultraschallsignal und Verarbeiten von jeglichem ungültigem Signal in dem ursprünglichen Ultraschallsignal, um ein Ultraschallsignal zu erhalten, das verarbeitet werden soll;
Extrahieren von Spitzenmerkmalen basierend auf dem Ultraschallsignal, das verarbeitet werden soll, und Bestimmen von Zielgewinnen, die den Spitzenmerkmalen entsprechen;
Durchführen von Gewinnverarbeitung an dem ursprünglichen Ultraschallsignal basierend auf den Zielgewinnen, um ein Zielultraschallsignal zu bestimmen;
Analysieren des Zielultraschallsignals, um die fetale Herzfrequenz zu bestimmen.

**14.** Erfassungsverfahren nach Anspruch 9, **dadurch gekennzeichnet, dass**, wenn die fetale Erfassungseinheit (20) eine Ultraschallerfassungseinheit und eine Blutsauerstoff-Erfassungseinheit umfasst, der Schritt des Erfassens von physiologischen Daten des Fötus durch die fetale Erfassungseinheit (20) Folgendes umfasst:

Erfassen eines fetalen Herzfrequenzsignals;
wenn bestätigt ist, dass das erfasste fetale Herzfrequenzsignal ein gültiges fetales Herzfrequenzsignal ist, Durchführen von Zugriffserfassung einer Gliedmaße;
wenn erfasst wird, dass auf eine Gliedmaße zugegriffen wird, Starten einer Blutsauerstoff-Erfassungsfunktion, um Blutsauerstofferfassung durchzuführen, um die physiologischen Daten des Fötus zu erhalten.

## Revendications

**1.** Doppler fœtal, comprenant :

un boîtier (1),
une unité de détection fœtale (20) et une unité de détection de paramètre maternel (30), lesdites unité de détection fœtale (20) et unité de détection de paramètre maternel (30) servant respectivement à recueillir des données physiologiques fœtales et des données physiologiques maternelles,
un organe de commande (7) agencé dans une cavité interne du boîtier (1),
l'organe de commande (7) servant à analyser les données physiologiques fœtales et les données physiologiques maternelles recueillies pour obtenir une fréquence cardiaque fœtale et un paramètre maternel ;
**caractérisé en ce que**
l'unité de détection fœtale (20) comprend une unité d'acquisition échographique (2), et l'unité d'acquisition échographique (2) comprend un composant à plaquettes (21) ayant une pluralité de plaquettes et un composant d'ajustement d'angle (22) permettant d'ajuster la direction de transmission de signal entre chacune des plaquettes et un corps cible ;
l'organe de commande (7) est relié au composant d'ajustement d'angle (22), et l'organe de commande (7) sert à commander l'action du composant d'ajustement d'angle (22) compte tenu de l'intensité de signal correspondant à chacune des plaquettes pour réaliser un suivi cible.

2. Doppler fœtal selon la revendication 1, **caractérisé en ce que**

l'unité de détection de paramètre maternel (30) est une unité d'acquisition d'oxygène du sang, les données physiologiques maternelles sont un signal d'oxygène du sang, et lorsque l'organe de commande (7) sert à analyser les données physiologiques maternelles recueillies pour obtenir le paramètre maternel, l'organe de commande (7) sert en outre à : déterminer une enveloppe du signal d'oxygène du sang et obtenir le paramètre maternel compte tenu de l'enveloppe ;
ou bien
l'unité de détection de paramètre maternel (30) est une plaquette en céramique piézoélectrique, les données physiologiques maternelles sont un signal d'écho ultrasonore, et lorsque l'organe de commande (7) sert à analyser les données physiologiques maternelles recueillies pour obtenir le paramètre maternel, l'organe de commande (7) sert en outre à : acquérir un signal d'enveloppe de fréquence maximale dans le signal d'écho ultrasonore, et obtenir le paramètre maternel compte tenu du signal d'enveloppe de fréquence maximale ;
ou bien
l'unité de détection de paramètre maternel (30) est une électrode ECG, les données physiologiques maternelles sont un signal ECG, et lorsque l'organe de commande (7) sert à analyser les données physiologiques maternelles recueillies pour obtenir le paramètre maternel, l'organe de commande (7) sert en outre à : analyser le signal ECG pour obtenir le paramètre maternel.

3. Doppler fœtal selon la revendication 1, **caractérisé en ce que** l'unité de détection de paramètre maternel (30) comprend une unité d'acquisition d'oxygène du sang (3), l'unité d'acquisition d'oxygène du sang (3) étant équipée d'une structure de guidage de lumière installée sur le boîtier (1) ; le boîtier (1) est doté d'une fenêtre de transmission de la lumière (111) ; l'unité d'acquisition d'oxygène du sang comprend un élément émetteur de lumière (31) et un élément receveur de lumière (32) agencés sur l'organe de commande (7), et la structure de guidage de lumière comprend un premier élément de guidage de lumière installé sur le boîtier (1) et situé sur un trajet de transmission de lumière entre l'élément émetteur de lumière (31) et la fenêtre de transmission de lumière (111) ; le premier élément de guidage de lumière étant conçu pour transmettre la lumière émise par l'élément émetteur de lumière (31) vers l'extérieur de la fenêtre de transmission de lumière (111) après qu'elle a été propagée par le premier élément de guidage de lumière ; la structure de guidage de lumière comprend en outre une première barrière agencée dans le boîtier (1) et située entre la fenêtre de transmission de lumière (111) et l'organe de commande (7), un premier canal de trajet optique étant prévu dans la première barrière, le premier élément de guidage de lumière étant une première colonne de guidage de lumière (33) agencée dans le premier canal de trajet optique.

4. Doppler fœtal selon la revendication 3, **caractérisé en ce que** la structure de guidage de lumière comprend en outre un deuxième élément de guidage de lumière agencé dans le boîtier (1) et situé sur un trajet de transmission de la lumière entre l'élément receveur de lumière (32) et la fenêtre de transmission de lumière (111), et le deuxième élément de guidage de lumière est conçu pour transmettre la lumière en incidence depuis la fenêtre de transmission de lumière (111) vers l'élément récepteur de lumière (32) après qu'elle a été propagée par le deuxième élément de guidage de lumière ; la structure de guidage de lumière comprend en outre une deuxième barrière agencée dans le boîtier (1) et située entre la fenêtre de transmission de lumière (111) et l'organe de commande (7), un deuxième canal de trajet optique est prévu dans la deuxième barrière, et le deuxième élément de guidage de lumière est une deuxième colonne de guidage de lumière (34) agencée dans le deuxième canal de trajet optique.

5. Doppler fœtal selon la revendication 4, **caractérisé en ce que** la première barrière et la deuxième barrière sont formées d'un seul tenant par un socle de blocage (35), le premier canal de trajet optique est un premier orifice traversant prévu dans le socle de blocage (35), et le deuxième canal de trajet optique est un deuxième orifice traversant prévu dans le socle de blocage (35).

**6.** Doppler fœtal selon la revendication 3, **caractérisé en ce que** le premier élément de guidage de lumière est une lentille condensatrice capable de faire converger la lumière en un faisceau lumineux, la lentille condensatrice comprend au moins une lentille convexe, et une surface de la lentille convexe sur un côté orienté vers l'élément émetteur de lumière (31) est une surface convexe.

**7.** Doppler fœtal selon l'une quelconque des revendications 3 à 6, **caractérisé en ce qu'**une surface d'acquisition de fréquence cardiaque fœtale de l'unité d'acquisition échographique (2) et une surface d'acquisition d'oxygène du sang de l'unité d'acquisition d'oxygène du sang sont situées sur deux surfaces différentes du boîtier (1) ; l'unité d'acquisition d'oxygène du sang (3) comprend un module de mesure de l'oxygène du sang destinée à acquérir un signal d'oxygène du sang et un module de détection de déclenchement agencé sur la surface d'acquisition d'oxygène du sang et relié électriquement à l'organe de commande ; lorsque le module de détection de déclenchement détecte la présence d'un objet obstruant près de la surface d'acquisition d'oxygène du sang, un signal de commande servant à commander la mise en marche du module de mesure d'oxygène du sang est envoyé à l'organe de commande.

**8.** Doppler fœtal selon la revendication 1, **caractérisé en ce que**
la pluralité de plaquettes est divisée en plaquettes centrales (211) et plaquettes périphériques (212), les plaquettes périphériques (212) sont agencées autour d'une périphérie des plaquettes centrales (211), l'organe de commande (7) servant à commander l'action du composant d'ajustement d'angle (22) compte tenu de l'intensité du signal correspondant aux plaquettes centrales (211) et de l'intensité du signal correspondant aux plaquettes périphériques (212).

**9.** Procédé de détection propre à un Doppler fœtal, dans lequel le Doppler fœtal comprend une unité de détection fœtale (20) et une unité de détection de paramètre maternel (30), le procédé de détection comprenant :

l'acquisition de données physiologiques fœtales recueillies par l'unité de détection fœtale (20) et de données physiologiques maternelles recueillies par l'unité de détection de paramètre maternel (30),
l'analyse des données physiologiques fœtales et des données physiologiques maternelles pour obtenir une fréquence cardiaque fœtale et un paramètre maternel ;
**caractérisé en ce que**

l'unité de détection fœtale (20) comprend une unité d'acquisition échographique (2), l'unité d'acquisition échographique (2) comprenant une pluralité de plaquettes et un composant d'ajustement d'angle (22) ; l'étape d'acquisition des données physiologiques fœtales recueillies par l'unité de détection fœtale (20) comprend :

l'acquisition d'une intensité de signal correspondant à chacune des plaquettes,
compte tenu de l'intensité du signal correspondant à chacune des plaquettes, la commande d'une action du composant d'ajustement d'angle (22) pour réaliser un suivi cible, ledit composant d'ajustement d'angle (22) servant à ajuster la direction de transmission de signal entre chacune des plaquettes et un corps cible,
l'acquisition des données physiologiques fœtales recueillies par chacune des plaquettes selon la direction de transmission de signal ajustée.

**10.** Procédé de détection selon la revendication 9, **caractérisé en ce que** le paramètre maternel comprend la fréquence cardiaque maternelle, le procédé de détection comprenant en outre :

la détection de la présence d'un intervalle de superposition entre la fréquence cardiaque fœtale et la fréquence cardiaque maternelle,
s'il est détecté un intervalle de superposition, l'envoi d'une requête et/ou le marquage de l'intervalle de superposition sur une courbe de fréquence cardiaque affichée, ladite courbe de fréquence cardiaque comprenant au moins l'une des suivantes : une courbe de fréquence cardiaque fœtale générée à partir de la fréquence cardiaque fœtale et une courbe de fréquence cardiaque maternelle générée à partir de la fréquence cardiaque maternelle.

**11.** Procédé de détection selon la revendication 10, **caractérisé en ce que** l'étape de détection de la présence d'intervalles de superposition entre la fréquence cardiaque fœtale et la fréquence cardiaque maternelle comprend :

l'acquisition d'un premier nombre de différences de fréquences cardiaques correspondant respectivement à différents moments d'une première période, ladite différence de fréquences cardiaques correspondant à chaque moment consistant en une valeur de différence caractéristique entre la fréquence cardiaque fœtale et la fréquence cardiaque maternelle à ce moment,
le comptage d'un deuxième nombre de différences de fréquences cardiaques remplissant une première condition au cours de la première période, la première condition étant que la différence de fréquences cardiaques soit inférieure à un seuil prédéfini,
si le deuxième nombre remplit une deuxième condition et que les différences de fréquences cardiaques relevées au cours d'une deuxième période incluse dans la première période remplissent toutes la première condition, la détermination du fait que la première période est un intervalle de superposition entre la fréquence cardiaque fœtale et la fréquence cardiaque maternelle, la deuxième condition étant que la proportion du deuxième nombre dans le premier nombre est supérieure à une proportion prédéfinie.

**12.** Procédé de détection selon la revendication 9, **caractérisé en ce que** la pluralité de plaquettes est divisée en plaquettes centrales et plaquettes périphériques, les plaquettes périphériques étant agencées autour d'une périphérie des plaquettes centrales, l'étape de commande de l'action du composant d'ajustement d'angle (22), selon l'intensité de signal correspondant à chacune des plaquettes, pour réaliser le suivi cible comprenant :

la comparaison de l'intensité de signal de la pluralité de plaquettes, et l'établissement du fait qu'une plaquette présentant la plus forte intensité de signal est une plaquette centrale ou non,
lorsque la plaquette ayant la plus forte intensité de signal est une plaquette périphérique, le calcul d'une valeur de différence d'intensités de signal entre l'intensité de signal des plaquettes centrales et la plus forte intensité de signal,
compte tenu de la valeur de différence d'intensités de signal et de la position de la plaquette ayant la plus forte intensité de signal, la détermination d'un angle d'ajustement et de la direction du composant d'angle d'ajustement (22).

**13.** Procédé de détection selon l'une quelconque des revendications 9 à 12, **caractérisé en ce que**, lorsque les données physiologiques consistent en un signal échographique original, l'étape d'analyse des données physiologiques fœtales recueillies pour obtenir une fréquence cardiaque fœtale comprend :

la réalisation d'une identification de signal valide sur le signal échographique original, et le traitement de tout signal invalide dans le signal échographique original pour obtenir un signal échographique à traiter,
l'extraction de caractéristiques de pic compte tenu du signal échographique à traiter, et la détermination de gains cibles correspondant aux caractéristiques de pic,
la réalisation d'un traitement des gains dans le signal échographique original compte tenu des gains cibles pour déterminer un signal échographique cible,
l'analyse du signal échographique cible pour déterminer la fréquence cardiaque fœtale.

**14.** Procédé de détection selon la revendication 9, **caractérisé en ce que**, lorsque l'unité de détection fœtale (20) comprend une unité d'acquisition échographique et une unité d'acquisition d'oxygène du sang, l'étape d'acquisition des données physiologiques fœtales recueillies par l'unité de détection fœtale (20) comprend :

l'acquisition d'un signal de fréquence cardiaque fœtale,
lorsqu'il est confirmé que le signal de fréquence cardiaque fœtale acquis est un signal de fréquence cardiaque fœtale valide, la réalisation d'une détection d'accès à un membre,
lorsqu'il est détecté que l'on a accédé à un membre, le lancement d'une fonction de détection d'oxygène du sang pour réaliser une détection d'oxygène du sang pour obtenir les données physiologiques fœtales.

10
20
Fetal detection unit
7
30
controller
Maternal parameter detection unit

Figure 1

4
1042
104
3
1041
1
105
102
103
106
6
101

Figure 2

104
A
7
9
5
102
8
6
2
101

Figure 3

104
34
111
33
11
1
35
7
36
32
31
A

Figure 4

5
102
6
101

Figure 5

21
Wafer component
22
Angle adjustment component
7
Controller

Figure 6

213
211
212
Figure 7a
213
211
212
Figure 7b
21
Wafer component
22
7
23
Angle adjustment component
Controller
Signal acquisition unit
24
Adjustment angle recognition unit

Figure 8

Z
Y
a
X

Figure 9

Transmission frequency signal
Ultrasonic transmitting unit
25
Ultrasonic transducer
Ultrasonic receiving unit
26
Frequency conversion circuit 261
Control signal

Figure 10

Amplitude
Frequency

Figure 11

Amplitude
f0
f1
f2
Frequency

Figure 12

C
R
Vc
L
VD

Figure 13

L
C1
C2
C3
Vc

Figure 14

Transmission frequency signal
25
Ultrasonic transmitting unit
Ultrasonic transducer
26
Ultrasonic receiving unit
Frequency conversion circuit 261
7
Controller
40
Human-computer interaction unit
Control signal

Figure 15

Transmission frequency signal
25
Ultrasonic transmitting unit
Ultrasonic transducer
26
Ultrasonic receiving unit
Frequency conversion circuit 261
50
Signal extraction unit
7
Controller
40
Human-computer interaction unit
Control signal

Figure 16

101
214
216

Figure 17

101
215
214
217

Figure 18

f1
f2
(a)
f1
f2
(b)
f1
f2
(c)
f1
f2
(d)
f1
f2
f3
(e)
f3
f1
f2
(f)
f1
f2
f3
(g)
f1
f2
f4
f3
(h)
(i)
(j)
(k)
f1
f2
f5
f4
f3
(l)
(m)
(n)

Figure 19

Amplitude
f0
Frequency

Figure 20

Amplitude
f0
f1
f2
Frequency

Figure 21

Amplitude
f0
f1
f2
f3
f4
Frequency

Figure 22

Acquiring fetal physiological data collected by the fetal detection unit and maternal physiological data collected by the maternal parameter detection unit
S11
Analyzing the collected fetal physiological data and maternal physiological data to obtain a fetal heart rate and a maternal parameter
S12

Figure 23

Acquiring a signal strength corresponding to each of the wafers
S21
Based on the signal strength corresponding to each of the wafers, controlling an action of the angle adjustment component to realize target tracking
S22
Acquiring the fetal physiological data collected by each of the wafers based on the adjusted signal transmission direction
S23

Figure 24

Performing valid signal identification on the original ultrasonic signal, and processing any invalid signal in the original ultrasonic signal to obtain an ultrasonic signal to be processed
S31
Extracting peak features based on the ultrasonic signal to be processed, and determining target gains corresponding to the peak features
S32
Performing gain processing on the original ultrasonic signal based on the target gains to determine a target ultrasonic signal
S33
Analyzing the target ultrasonic signal to determine the fetal heart rate
S34

Figure 25

Original signal
Signal after gain-control

Figure 26a

Original signal
Signal after gain-control

Figure 26b

Base-noise original signal
Signal after intelligent noise reduction

Figure 26c

S41
Acquiring a fetal heart-rate signal
S42
When it is confirmed that the fetal heart-rate signal acquired is a valid fetal heart-rate signal, performing limb access detection
S43
When it is detected that a limb is accessed, starting a blood oxygen detection function to perform blood oxygen detection to obtain the fetal physiological data

Figure 27

T1
T2
TH
TL
S1
T3
T4

Figure 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20180317878 A1